(19) Europäisches Patentamt
European Patent Office
Office européen des brevets

(11) EP 1 580 204 A1

(12) **DEMANDE DE BREVET EUROPEEN**

(43) Date de publication:
**28.09.2005 Bulletin 2005/39**

(51) Int Cl.[7]: **C08F 293/00**, C08F 220/30, C08F 220/38

(21) Numéro de dépôt: **05290197.2**

(22) Date de dépôt: **28.01.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **26.03.2004 FR 0403183**

(71) Demandeur: **L'OREAL**
**75008 Paris (FR)**

(72) Inventeurs:
- **Luukas, Timo**
  **91300 Massy (FR)**
- **Farcet, Céline**
  **75010 Paris (FR)**

(74) Mandataire: **Dodin, Catherine**
**L'OREAL - D.I.P.I.**
**25-29 Quai Aulagnier**
**92600 Asnières (FR)**

(54) **Nouveaux polymères séquencés, compositions les comprenant, procédés et utilisation**

(57) La présente demande concerne de nouveaux polymères séquencés comprenant au moins un monomère à effet optique, les compositions cosmétiques les comprenant, notamment les compositions de vernis à ongles, de fond de teint et les compositions anti-age.

L'invention concerne également un procédé de traitement cosmétique employant ladite composition, et l'utilisation de ces polymères comme tenseurs.

EP 1 580 204 A1

## Description

La présente invention a trait à de nouveaux polymères de structure spécifique ainsi qu'aux compositions cosmétiques comprenant de tels polymères. L'invention concerne également un procédé de traitement cosmétique employant lesdits polymères.

Divers types de polymères sont conventionnellement utilisés dans les compositions cosmétiques en raison des diverses propriétés qu'ils peuvent leur apporter. Ils sont par exemple utilisés dans des compositions de maquillage ou de soin de la peau, des lèvres ou des phanères, telles que des vernis à ongles ou des compositions pour les cheveux. Cependant, en utilisant au sein d'une même composition deux polymères incompatibles, c'est à dire non miscibles dans un même solvant, le formulateur est confronté, du fait de l'incompatibilité des polymères, à des problèmes de séparation de phases, voire de décantation, et de manière générale à l'obtention d'une composition non homogène. Ces problèmes ne pouvaient être jusqu'ici résolus que par la présence dans la composition d'un composé permettant de compatibiliser les polymères entre eux.

Le but de la présente invention est de proposer un polymère qui, lorsqu'il est inclus dans une composition, en particulier une composition cosmétique, permette que cette composition ne présente pas les inconvénients, limitations, défauts et désavantages des compositions de l'art antérieur.
Ce but est atteint, conformément à la présente invention, grâce à un polymère, dit polymère séquencé, comprenant au moins une première séquence et au moins une deuxième séquence incompatibles l'une avec l'autre et ayant des températures de transition vitreuse (Tg) de préférence différentes, lesdites première et deuxième séquences étant reliées entre elles par un segment intermédiaire comprenant au moins un monomère constitutif de la première séquence et au moins un monomère constitutif de la deuxième séquence.

Un objet de la présente invention est donc un polymère tel que défini ci-après.

L'invention a également pour objet une composition notamment cosmétique comprenant ledit polymère.

On a constaté que les polymères selon l'invention présentent de bonnes propriétés optiques qui les rendent susceptibles d'être employés en cosmétique, permettant d'obtenir des effets optiques adéquats des compositions les comprenant et/ou du maquillage obtenu à l'aide de ces compositions.
Ils peuvent présenter, selon la nature des substituants, une variabilité de couleur importante, pouvant aller du bleu/violet jusqu'à l'orange/rouge, en passant par le jaune. Ceci permet de disposer d'une gamme de composés, appartenant à la même famille chimique et donc se formulant de manière similaire, qui propose une diversité de propriétés optiques; ceci facilite notamment le travail des formulateurs en leur permettant de garder une architecture commune à l'ensemble de leurs compositions, quel que soit les polymères à propriété optique employés.

Par ailleurs, les polymères selon l'invention présentent de bonnes propriétés de fluorescence, et dans certains d'entre eux, d'azurant optique.
On rappelle que les composés fluorescents absorbent dans l'ultraviolet et dans le visible, et réémettent de l'énergie par fluorescence pour une longueur d'onde comprise entre 380 nm et 830 nm. Lorsque cette longueur d'ondes est comprise entre 380 nm et 480 nm, c'est-à-dire dans le bleu du domaine visible, les composés sont alors des azurants optiques.

Les polymères selon l'invention peuvent se présenter sous forme solide ou liquide, et confèrent des effets optiques remarquables aux compositions qui les comprennent ainsi qu'au maquillage déposé; en particulier, ils peuvent apporter des effets éclaircissants, des effets illuminants et/ou des effets de couleur.
Par ailleurs, ces polymères présentent une bonne stabilité à la température, au pH et à la lumière.
On a également constaté que les polymères selon l'invention présentent une bonne solubilité dans les corps gras, solubilité qui peut varier et être ajustée, selon la nature des monomères. Cette bonne liposolubilité peut également faciliter leur mise en oeuvre ultérieure, notamment dans les compositions cosmétiques qui comprennent généralement une phase grasse.

Par "au moins" une séquence, on entend une ou plusieurs séquences.

Par "séquences incompatibles l'une avec l'autre", on entend que le mélange formé du polymère correspondant à la première séquence et du polymère correspondant à la deuxième séquence, n'est pas miscible dans le solvant de polymérisation majoritaire en poids du polymère séquencé, à température ambiante (25°C) et pression atmosphérique ($10^5$ Pa), pour une teneur du mélange de polymères supérieure ou égale à 5 % en poids, par rapport au poids total du mélange (polymères et solvant), étant entendu que :

i) lesdits polymères sont présents dans le mélange en une teneur telle que le rapport pondéral respectif va de 10/90 à 90/10, et que
ii) chacun des polymères correspondant au première et seconde séquences a une masse moléculaire moyenne (en poids ou en nombre) égale à celle du polymère séquencé +/- 15%.

Dans le cas d'un mélange de solvants de polymérisation, dans l'hypothèse de deux ou plusieurs solvants présents,

ledit mélange de polymères est non miscible dans au moins l'un d'entre eux.
Bien entendu, dans le cas d'une polymérisation réalisée dans un solvant unique, ce dernier est le solvant majoritaire.

Le segment intermédiaire est une séquence comprenant au moins un monomère m1 constitutif de la première séquence et au moins un monomère m2 constitutif de la deuxième séquence du polymère; de préférence m2 est différent de m1. Le segment ou séquence intermédiaire peut permettre de "compatibiliser" ces première et deuxième séquences.

En incorporant ces nouveaux polymères dans des compositions cosmétiques, la demanderesse a découvert que certains de ces polymères décrits plus en détail ci-dessous, avaient des propriétés cosmétiques très intéressantes.

De manière générale, ces polymères peuvent augmenter la résistance aux chocs des vernis à ongles et améliorent la tenue d'une grande variété de compositions de maquillage, notamment fond de teint ou rouge à lèvres, sans provoquer chez l'utilisatrice de sentiment d'inconfort. Ils peuvent par ailleurs présenter des propriétés de tenseur.

Le polymère séquencé de la composition selon l'invention est avantageusement un polymère éthylénique séquencé linéaire, de préférence formant un dépôt, et plus particulièrement filmogène.
Par polymère "éthylénique" , on entend un polymère obtenu par polymérisation de monomères comprenant une insaturation éthylénique.
Par polymère "séquencé", on entend un polymère comprenant au moins 2 séquences distinctes, de préférence au moins 3 séquences distinctes.
Le polymère est un polymère à structure linéaire. Par opposition, un polymère ayant une structure non linéaire est, par exemple, un polymère à structure ramifiée, en étoile, greffée, ou autre.
Par polymère "formant un dépôt", on entend un polymère apte à former à lui seul ou en présence d'un agent auxiliaire, un dépôt adhérent sur un support, notamment sur les matières kératiniques.
Par polymère "filmogène", on entend un polymère apte à former à lui seul ou en présence d'un agent auxiliaire de filmification, un film continu et adhérent sur un support, notamment sur les matières kératiniques.

Le polymère selon l'invention comprend au moins une première séquence (ou bloc) et au moins une deuxième séquence (ou bloc) incompatibles l'une avec l'autre et ayant de préférence des températures de transition vitreuse (Tg) différentes, lesdites première et deuxième séquences étant reliées entre elles par un segment intermédiaire comprenant au moins un monomère constitutif de la première séquence et au moins un monomère constitutif de la deuxième séquence.

On précise que dans ce qui précède et ce qui suit les termes "première" et "deuxième" séquences ne conditionnent nullement l'ordre desdites séquences (ou blocs) dans la structure du polymère.

Chaque séquence, ou bloc, du polymère selon l'invention est issue d'un type de monomère ou de plusieurs types de monomères différents. Cela signifie que chaque séquence peut être constituée d'un homopolymère ou d'un copolymère; ce copolymère constituant la séquence pouvant être à son tour statistique ou alterné.
Avantageusement, le segment intermédiaire comprenant au moins un monomère constitutif de la première séquence et au moins un monomère constitutif de la deuxième séquence du polymère est un polymère statistique. De préférence, la séquence intermédiaire est issue essentiellement de monomères constitutifs de la première séquence et de la deuxième séquence. Par "essentiellement", on entend au moins à 85%, de préférence au moins à 90%, mieux à 95% et encore mieux à 100%.

Avantageusement, la séquence intermédiaire a une température de transition vitreuse Tg comprise entre les températures de transition vitreuse des première et deuxième séquences.
Selon l'invention, les première et deuxième séquences ont de préférence des températures de transition vitreuse différentes, avec un écart entre les températures de transition vitreuse des première et deuxième séquences généralement supérieur à 5°C, de préférence supérieur à 10°C, et mieux supérieur à 20°C.
Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$\frac{1}{Tg} = \sum_i \left(\frac{\varpi i}{Tgi}\right)$$

wi étant la fraction massique du monomère i dans la séquence considérée et Tgi étant la température de transition vitreuse de l'homopolymère du monomère i.

Sauf indication contraire, les Tg indiquées pour les première et deuxième séquences dans la présente demande sont des Tg théoriques.

Dans tous les cas, au moins l'une des séquences comprend un monomère ci-après appelé à effet optique', qui est de formule (I) :

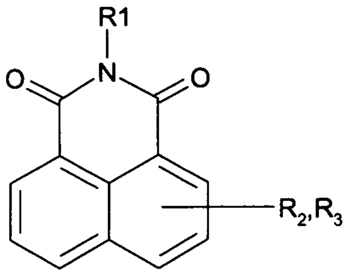

(I)

dans laquelle :

- R1 représente un radical carboné linéaire, ramifié et/ou cyclique, saturé et/ou insaturé, comprenant 1 à 32 atomes de carbone; éventuellement substitué par un ou plusieurs groupements choisis parmi =O, OH, $NH_2$, et les atomes d'halogènes; et/ou éventuellement interrompu par un ou plusieurs hétéroatomes choisis parmi O, N, P, Si et S;

- R2 et R3, présents sur le même cycle ou chacun sur un cycle différent, représentent, indépendamment l'un de l'autre, un hydrogène, un halogène, ou un groupement de formule -X-G-P (II), sous réserve qu'au moins l'un des radicaux R2 et/ou R3 représente un groupement de formule (II), dans laquelle :

- X est choisi parmi les groupements -O-, -S-, -SO-, $-SO_2-$, -NH- ou -NR4- avec R4 représentant un radical carboné linéaire, ramifié et/ou cyclique, saturé et/ou insaturé, comprenant 1 à 30 atomes de carbone, éventuellement substitué par un ou plusieurs groupements choisis parmi =O, OH, $NH_2$, et les atomes d'halogènes; et/ou éventuellement interrompu par un ou plusieurs hétéroatomes choisis parmi O, N, P, Si et S;

- G est un radical carboné divalent linéaire, ramifié et/ou cyclique, saturé et/ou insaturé, comprenant 1 à 32 atomes de carbone, éventuellement substitué par un ou plusieurs groupements choisis parmi =O, OH, $NH_2$, et les atomes d'halogènes; et/ou éventuellement interrompu par un ou plusieurs hétéroatomes choisis parmi O, N, P, Si et S;

- P est un groupement polymérisable choisi parmi l'une des formules suivantes :

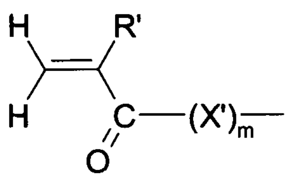

(IIIa)

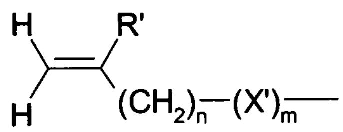

(IIIb)

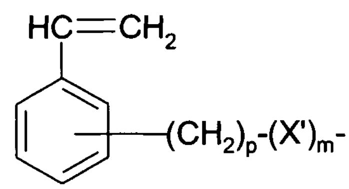

(IIIc)

dans lesquelles :

- R' représente H ou un radical hydrocarboné, linéaire ou ramifié, saturé en C1-6,
- X' représente O, NH ou NR" avec R" représentant un radical choisi parmi les radicaux alkyles en C1-6, aryles en C6-10, aryl(C6-10)alkyles(C1-6) ou alkyle(C1-6)aryles(C6-10), les groupements alkyle et/ou aryle pouvant en outre être substitués par un ou plusieurs groupements choisis parmi OH, halogène, alcoxy en C1-6 et aryloxy en C6-10; et
- m est égal à 0 ou 1; n est égal à 0 ou 1 et p est égal à 0, 1 ou 2.

Dans la présente invention, on entend par 'radical cyclique' un radical monocyclique ou polycyclique, qui se présente donc lui-même sous forme d'un ou plusieurs cycles, saturés et/ou insaturés, éventuellement substitués (par exemple cyclohexyle, cyclodécyle, benzyle ou fluorényle), mais également un radical qui comprend un ou plusieurs desdits cycles (par exemple p-tertbutylcyclohexyle ou 4-hydroxybenzyle).
Dans la présente invention, on entend par ' radical saturé et/ou insaturé', les radicaux totalement saturés, les

radicaux totalement insaturés, y compris aromatiques, ainsi que les radicaux comportant une ou plusieurs doubles et/ou triples liaisons, le reste des liaisons étant des liaisons simples.

De préférence, R1 peut notamment être un radical hydrocarboné cyclique, linéaire et/ou ramifié, saturé ou insaturé, comprenant éventuellement un cycle hydrocarboné lui-même saturé ou insaturé, comprenant 3 à 18, notamment 4 à 14, atomes de carbone, et peut comprendre au moins un hétéroatome, notamment un ou deux atomes d'azote, d'oxygène ou de silicium.

Préférentiellement, R1 peut être un radical hydrocarboné linéaire, ramifié et/ou cyclique, saturé ou insaturé, comprenant 6 à 13 atomes de carbone.

Notamment, R1 peut être un radical n-propyle, isopropyle, n-butyle, isobutyle, tert-butyle, n-pentyle, cyclopentyle, n-hexyle, cyclohexyle, n-heptyle, n-octyle, cyclooctyle, décyle, cyclodécyle, dodécyle, cyclododécyle, p-tertbutylcyclohexyle, benzyle, phényle, ou un radical choisi parmi l'éthyl-2-N-pyrrolidine, la (2-méthyl)-1-éthylpyrrolidine, le 3-propyltriéthoxysilane, les dialkylpyrimidines et notamment la 2-(4,6-diméthyl)-pyrimidine, le radical benzothiazyle, le radical fluorényle.

| | |
|---|---|
| 2-méthyl-1-ethylpyrrolidine | 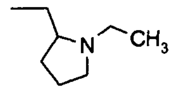 |
| éthyl-2-N-pyrrolidine | 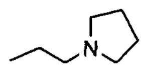 |
| 3-propyl-triethoxysilane | 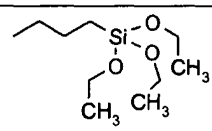 |
| benzothiazyle | 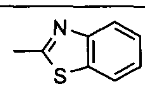 |
| fluorényle | 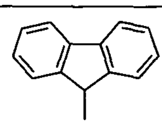 |
| 2-(4,6-dimethyl)pyrimidine | 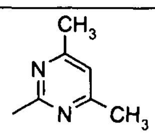 |

Le radical R2 est de préférence un atome d'hydrogène, et donc R3 un groupement de formule (II).

Dans ledit groupement de formule (II), X est de préférence choisi parmi -O-, -S-, -NH- ou -NR4-.
Le radical R4 représente préférentiellement un radical hydrocarboné linéaire, ramifié et/ou cyclique, saturé ou insaturé, comprenant éventuellement un cycle hydrocarboné lui-même saturé ou insaturé, comprenant 2 à 18, notamment 3 à 12 atomes de carbone, éventuellement substitué par un ou plusieurs groupements choisis parmi =O, OH, $NH_2$, et les atomes d'halogènes; et/ou éventuellement interrompu par un ou plusieurs hétéroatomes choisis parmi O, N, P, Si et S.
Notamment R4 peut être un radical éthyle, n-propyle, isopropyle, n-butyle, iso-butyle, tert-butyle, pentyle, hexyle, cyclohexyle, octyle, décyle, dodécyle, phényle ou benzyle.

Le radical divalent G est de préférence un radical hydrocarboné divalent linéaire, ramifié et/ou cyclique, saturé ou insaturé, comprenant éventuellement un cycle hydrocarboné lui-même saturé ou insaturé, comprenant au total 2 à 18, notamment 3 à 10 atomes de carbone, éventuellement substitué par un ou plusieurs groupements choisis parmi =O, OH, $NH_2$, et les atomes d'halogènes; et/ou éventuellement interrompu par un ou plusieurs hétéroatomes choisis parmi O, N, P, Si.
Préférentiellement G est choisi parmi les radicaux hydrocarbonés divalents linéaires ou ramifiés, saturés comprenant éventuellement un cycle hydrocarboné saturé, comprenant au total 2 à 18, notamment 3 à 10 atomes de carbone.
Ainsi G peut être choisi parmi les radicaux éthylène, n-propylène, isopropylène (ou méthyl-1 éthylène et méthyl-2 éthylène), n-butylène, isobutylène, pentylène notamment n-pentylène, hexylène notamment n-hexylène, heptylène, cyclohexylène, octylène, décylène, cyclohexyldiméthylène notamment de formule - $CH_2$-$C_6H_{10}$-$CH_2$-, dodécylène.

Dans la formule (IIIb), si n =0 alors de préférence, m=0.

Le groupement polymérisable P est de préférence choisi parmi l'une des formules suivantes :

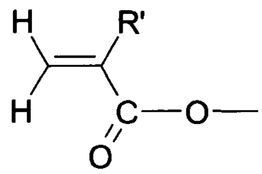
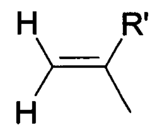

dans lesquelles R' représente H ou méthyle.

Parmi les composés monomériques particulièrement préférés selon l'invention, on peut citer les composés répondant à l'une des formules suivantes :

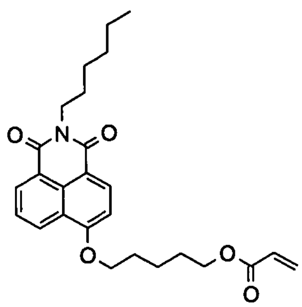
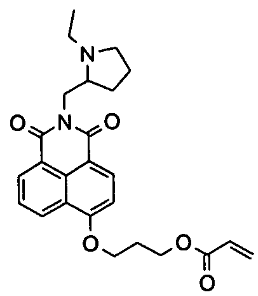
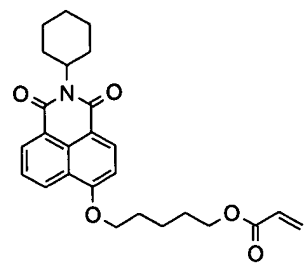
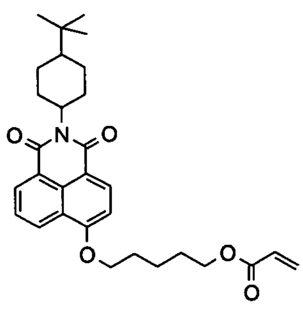
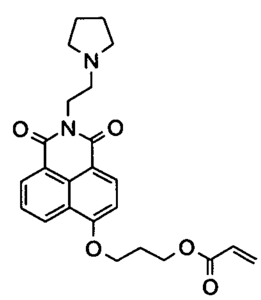
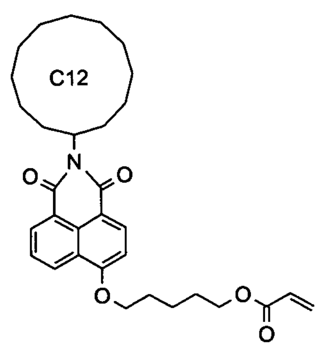
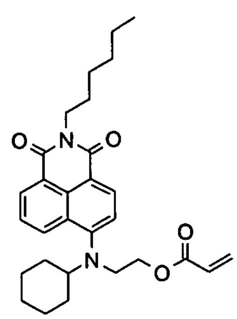
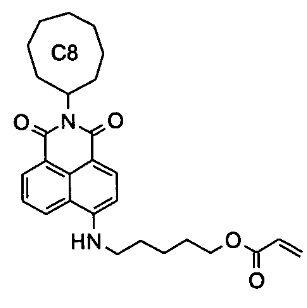
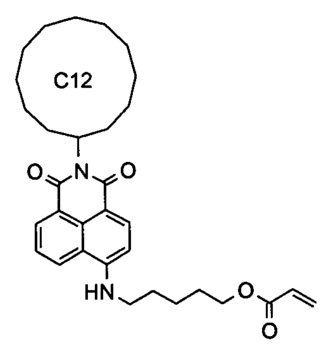

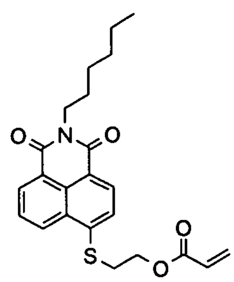

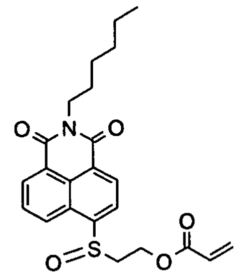

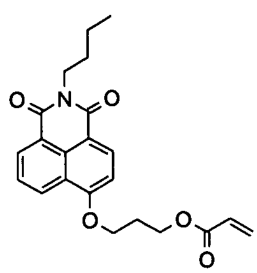

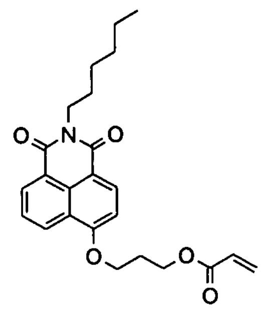

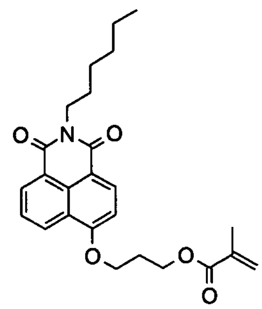

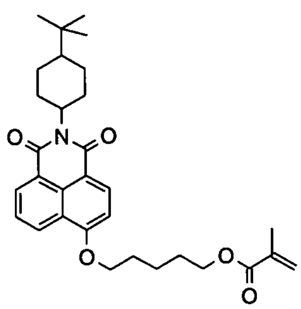

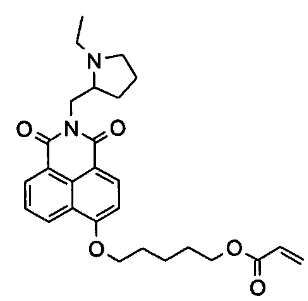

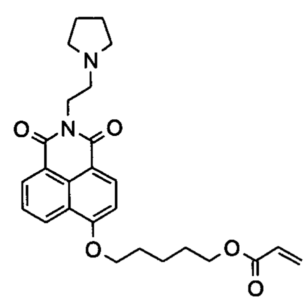

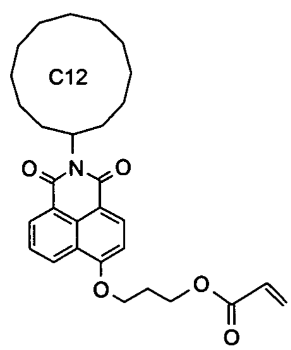

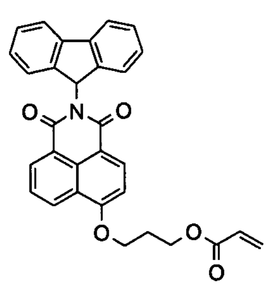

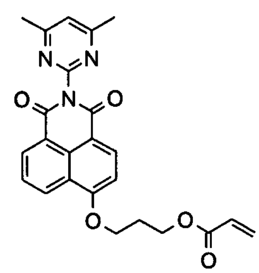

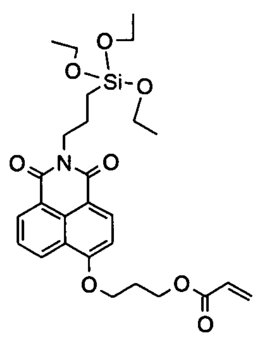
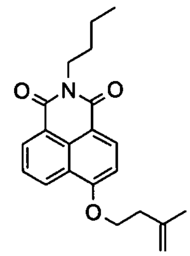
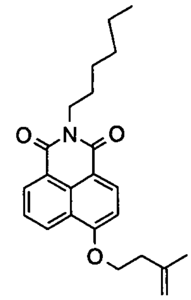

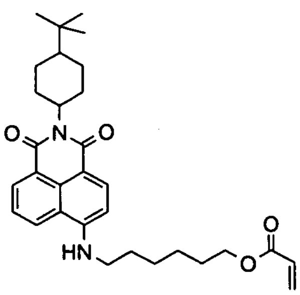
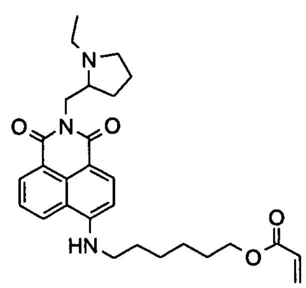
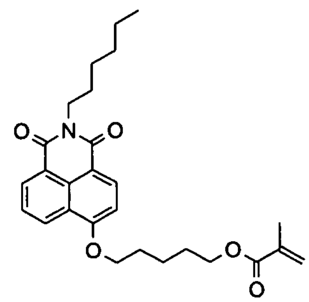

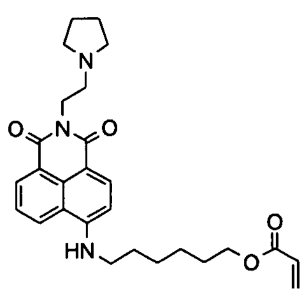
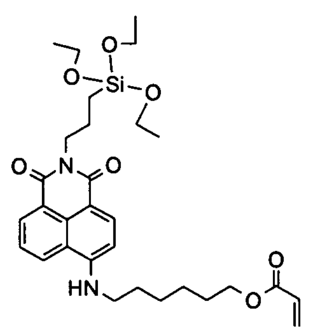
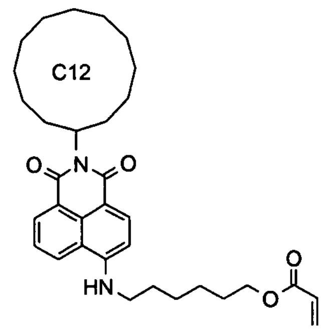

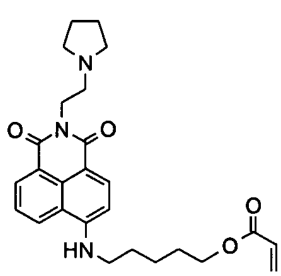
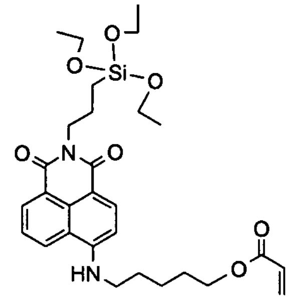
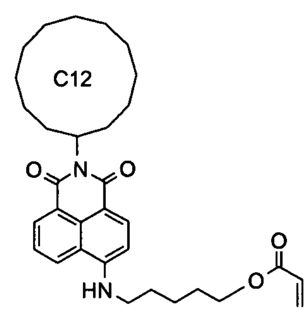

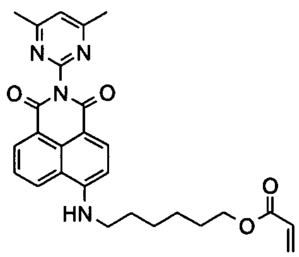

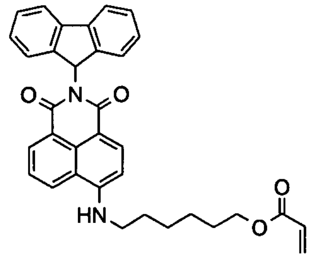

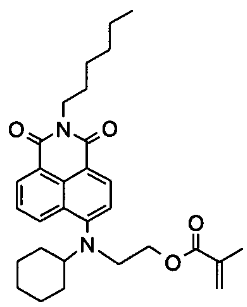

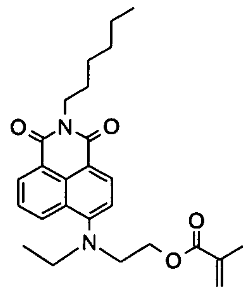

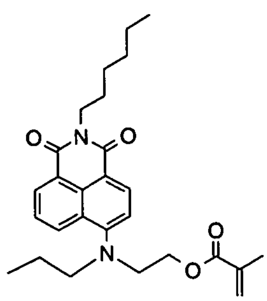

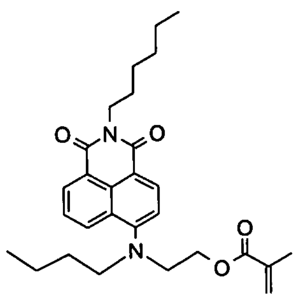

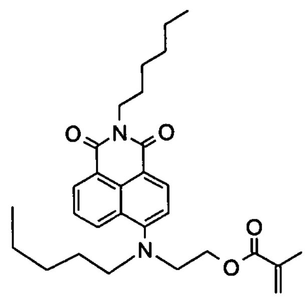

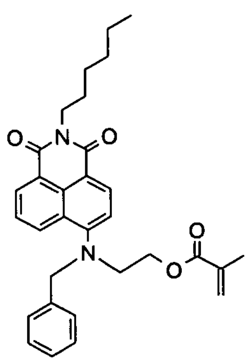

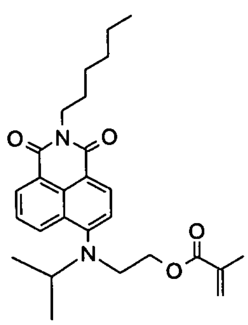

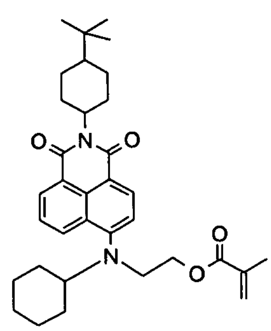

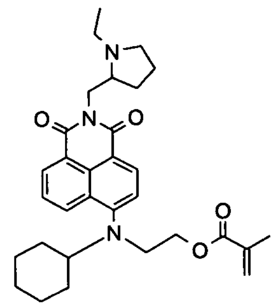
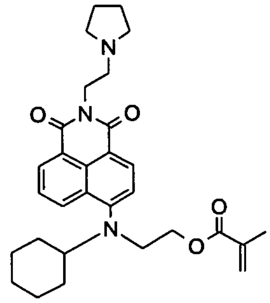
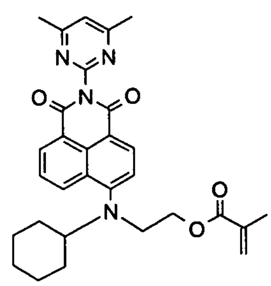

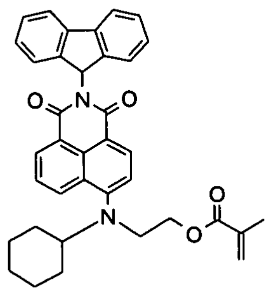
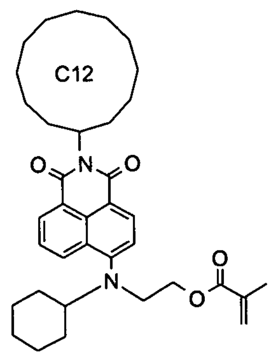

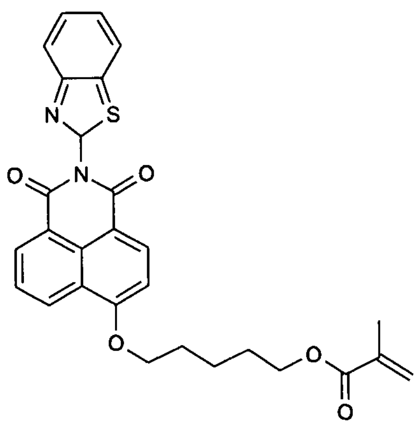
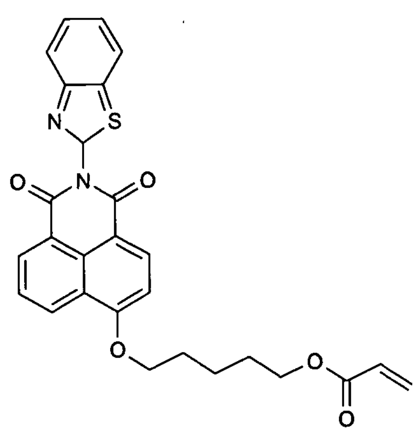

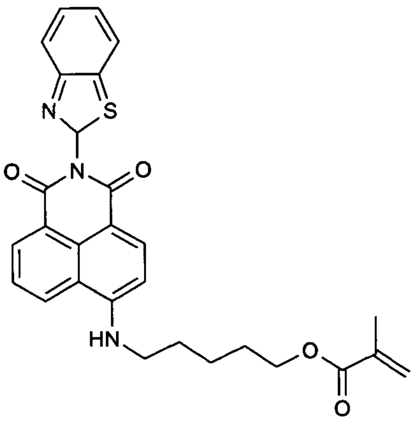

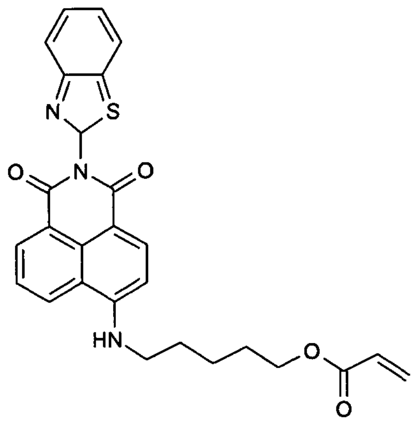

Ce monomère peut être présent, seul ou en mélange, dans la première et/ou la deuxième séquence. La séquence comprenant ledit monomère à effet optique de formule (I) peut donc être :

- (i) un homopolymère ne comprenant qu'un seul monomère à effet optique de formule (I),
- (ii) un copolymère comprenant plusieurs monomères à effet optique de formule (I),
- (iii) un copolymère comprenant un ou plusieurs monomères à effet optique de formule (I), et un ou plusieurs monomères additionnels, pouvant être choisis notamment parmi les monomères à effet optique de formule (A), (B) et/ou (C) ci-après et les monomères additionnels dits 'usuels'.

Parmi les monomères additionnels susceptibles d'être présents dans la séquence comprenant le ou les monomères à effet optique de formule (I), et/ou susceptibles d'être présents dans la ou les autres séquences ne comprenant pas de monomère à effet optique de formule (I), on peut citer, seul ou en mélange, les monomères suivants :

- (i) les hydrocarbures éthyléniques ayant 2 à 10 carbones, tels que l'éthylène, l'isoprène, ou le butadiène ;

- (ii) les (méth)acrylates de formule:

$$CH_2 = CHCOOR'_3$$

ou

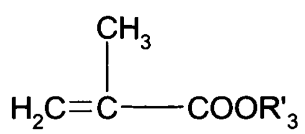

dans lesquelles $R'_3$ représente :

- un groupe alkyle linéaire ou ramifié, de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F), et les groupes Si ($R_4R_5$), où $R_4$ et $R_5$ identiques ou différents représentent un groupe alkyle en $C_1$ à $C_6$ ou un groupe phényle ;
  notamment R'3 peut être un groupe méthyle, éthyle, propyle, n-butyle, isobutyle, tertiobutyle, hexyle, éthylhexyle, octyle, lauryle, isooctyle, isodécyle, dodécyle, cyclohexyle, t-butylcyclohexyle ou stéaryle; éthyl-2-perfluorohexyle; ou un groupe hydroxyalkyle en $C_{1-4}$ tel que 2-hydroxyéthyle, 2-hydroxybutyle et 2-hydroxypropyle; ou un groupe alcoxy ($C_{1-4}$) alkyle ($C_{1-4}$) tel que méthoxyéthyle, éthoxyéthyle et méthoxypropyle,
- un groupe cycloalkyle en $C_3$ à $C_{12}$, tel que le groupe isobornyle,
- un groupe aryle en $C_3$ à $C_{20}$ tel que le groupe phényle,

- un groupe aralkyle en $C_4$ à $C_{30}$ (groupe alkyle en $C_1$ à $C_8$) tel que 2-phényl-éthyle, t-butylbenzyle ou benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle de 1 à 4 C), tel que furfurylméthyle ou tétrahydrofurfurylméthyle, lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle, les atomes d'halogène (Cl, Br, I et F), et les groupes Si ($R_4R_5$), où $R_4$ et $R_5$ identiques ou différents représentent un groupe alkyle en $C_1$ à $C_6$, ou un groupe phényle,
- $R'_3$ peut également être un groupe $-(C_2H_4O)_m-R''$, avec m = 5 à 150 et R" = H

ou alkyle de $C_1$ à $C_{30}$, par exemple -POE-méthyle ou -POE-béhényle;

- (iii) les (méth)acrylamides de formule :

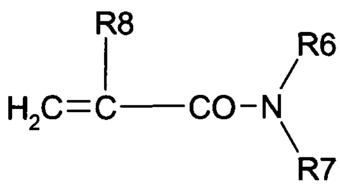

dans laquelle $R_8$ désigne H ou méthyle; et $R_7$ et $R_6$ identiques ou différents représentent :

- un atome d'hydrogène; ou
- un groupe alkyle linéaire ou ramifié, de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F), et les groupes Si ($R_4R_5$), où $R_4$ et $R_5$ identiques ou différents représentent un groupe alkyle en $C_1$ à $C_6$ ou un groupe phényle ;
  notamment R6 et/ou R7 peut être un groupe méthyle, éthyle, propyle, n-butyle, isobutyle, tertiobutyle, hexyle, éthylhexyle, octyle, lauryle, isooctyle, isodécyle, dodécyle, cyclohexyle, t-butylcyclohexyle ou stéaryle; éthyl-2-perfluorohexyle;
  ou un groupe hydroxyalkyle en $C_{1-4}$ tel que 2-hydroxyéthyle, 2-hydroxybutyle et 2-hydroxypropyle; ou un groupe alcoxy ($C_{1-4}$) alkyle ($C_{1-4}$) tel que méthoxyéthyle, éthoxyéthyle et méthoxypropyle,
- un groupe cycloalkyle en $C_3$ à $C_{12}$, tel que le groupe isobornyle,
- un groupe aryle en $C_3$ à $C_{20}$ tel que le groupe phényle,
- un groupe aralkyle en $C_4$ à $C_{30}$ (groupe alkyle en $C_1$ à $C_8$) tel que 2-phényl-éthyle, t-butylbenzyle ou benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle de 1 à 4 C), tel que furfurylméthyle ou tétrahydrofurfurylméthyle,

lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle, les atomes d'halogène (Cl, Br, I et F), et les groupes Si ($R_4R_5$), où $R_4$ et $R_5$ identiques ou différents représentent un groupe alkyle en $C_1$ à $C_6$, ou un groupe phényle.
Des exemples de monomères (méth)acrylamide sont le (méth)acrylamide, le N-éthyl(méth)acrylamide, le N-butylacrylamide, le N-t-butylacrylamide, le N-isopropylacrylamide, le N,N-diméthyl(méth)acrylamide, le N,N-dibutylacrylamide, le N-octylacrylamide, le N-dodécylacrylamide, l'undécylacrylamide, et le N(2-hydroxypropylméthacrylamide).

- (iv) les composés vinyliques de formules:

$$CH_2=CH-R_9,\ CH_2=CH-CH_2-R_9$$

ou

$$CH_2=C(CH_3)-CH_2-R_9$$

dans lesquelles $R_9$ est un groupe hydroxyle, halogène (Cl ou F), $NH_2$, $OR_{14}$ où $R_{14}$ représente un groupe phényle ou un groupe alkyle en $C_1$ à $C_{12}$ (le monomère est un éther de vinyle ou d'allyle); acétamide ($NHCOCH_3$); un groupe $OCOR_{15}$ où $R_{15}$ représente un groupe alkyle de 2 à 12 carbones, linéaire ou ramifié (le monomère est un ester de vinyle ou d'allyle); ou un groupe choisi parmi :

- un groupe alkyle linéaire ou ramifié, de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F), et les groupes Si ($R_4R_5$), où $R_4$ et $R_5$ identiques ou différents représentent un groupe alkyle en $C_1$ à $C_6$ ou un groupe phényle ;
- un groupe cycloalkyle en $C_3$ à $C_{12}$ tel que isobornyle, cyclohexane,
- un groupe aryle en $C_3$ à $C_{20}$ tel que phényle,
- un groupe aralkyle en $C_4$ à $C_{30}$ (groupe alkyle en $C_1$ à $C_8$) tel que 2-phényléthyle ; benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle de 1 à 4 C), tel que furfurylméthyle ou tétrahydrofurfurylméthyle,

lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle les atomes d'halogène (Cl, Br, I et F) et les groupes Si($R_4R_5$) où $R_4$ et $R_5$ identiques ou différents représentent un groupe alkyle en $C_1$ à $C_6$, ou un groupe phényle.
Des exemples de monomères vinyliques sont le vinylcyclohexane, et le styrène. Des exemples d'esters de vinyle sont l'acétate de vinyle le propionate de vinyle, le butyrate de vinyle, l'éthylhexanoate de vinyle, le néononanoate de vinyle et le néododécanoate de vinyle.
Parmi les éthers de vinyle, on peut citer le vinyl méthyl éther, le vinyl éthyl éther et le vinyl isobutyl éther.

- (v) les monomères (méth)acryliques, (méth)acrylamides ou vinyliques à groupe fluoré ou perfluoré, tels que le (méth)acrylate d'éthylperfluorooctyle ou d'éthyl-2-perfluorohexyle;

- (vi) les monomères (méth)acryliques, (méth)acrylamides ou vinyliques siliconés, tels que le méthacryloxypropyltris (triméthylsiloxy)silane ou l'acryloxypropylpolydiméthylsiloxane.

- (vii) les monomères à insaturation(s) éthylénique(s) comprenant au moins une fonction acide carboxylique, phosphorique ou sulfonique, ou anhydride, comme par exemple l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,

- (viii) les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci.

Les sels peuvent être formés par neutralisation des groupes anioniques à l'aide d'une base minérale, telle que LiOH, NaOH, KOH, $Ca(OH)_2$, $NH_4OH$ ou $Zn(OH)_2$; ou par une base organique telle qu'une alkylamine primaire, secondaire ou tertiaire, notamment la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la diméthylamino-2-propanol. On peut encore citer la lysine ou la 3-(diméthylamino)-propylamine.

On peut également peut citer les sels d'acides minéraux, tels que l'acide sulfurique, l'acide chlorhydrique, l'acide bromhydrique, l'acide iodhydrique, l'acide phosphorique, l'acide borique. On peut aussi citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

On choisit plus particulièrement les comonomères additionnels parmi, seuls ou en mélange, les (méth)acrylates d'alkyle en C1-C18 ou de cycloalkyle en C3-C12, et notamment parmi l'acrylate de méthyle, le méthacrylate de méthyle, l'acrylate d'isobornyle, le méthacrylate d'isobornyle, l'acrylate d'isobutyle, le méthacrylate d'isobutyle, l'acrylate d'éthyl-2-hexyle, le méthacrylate d'éthyl-2-hexyle, l'acrylate de dodécyle, le méthacrylate de dodécyle, l'acrylate de stéaryle, le méthacrylate de stéaryle, l'acrylate de trifluoroéthyle, le méthacrylate de trifluoroéthyle.

On peut également citer l'acide acrylique, l'acide méthacrylique, le méthacryloxypropyltris(triméthylsiloxy)silane, l'acryloxypropyltris(triméthylsiloxy)silane, l'acryloxypropylpolydiméthylsiloxane et le méthacryloxypropylpolydiméthylsiloxane.

Parmi les monomères additionnels susceptibles d'être présents dans la séquence comprenant le ou les monomères à effet optique de formule (I), et/ou susceptibles d'être présents dans la ou les autres séquences ne comprenant pas de monomère à effet optique de formule (I), on peut citer les monomères à effet optique de formule (A), (B) et/ou, (C) :

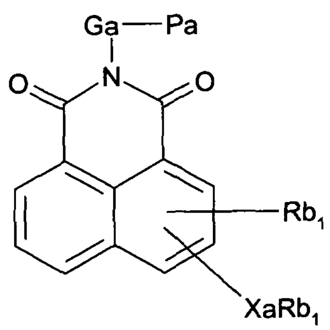

(A)

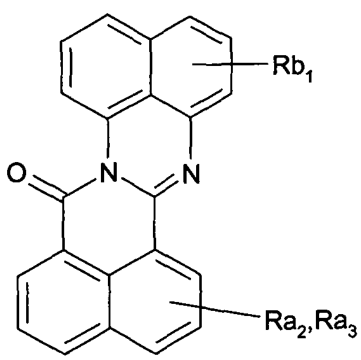

(B)

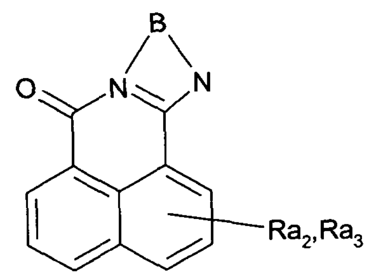

(C)

dans lesquelles :

- Rb1 est choisi parmi (i) un atome d'hydrogène, (ii) un halogène, (iii) un radical carboné linéaire, ramifié et/ou cyclique, saturé et/ou insaturé, comprenant 1 à 12 atomes de carbone, éventuellement substitué par un ou plusieurs groupements choisis parmi =O, OH, $NH_2$ et/ou éventuellement interrompu par un ou plusieurs hétéroatomes choisis parmi O, N, P, Si et S; (iv) un groupement NRR' avec R et R' étant, indépendamment 'un de l'autre, un atome d'hydrogène ou un radical hydrocarboné, linéaire, cyclique ou ramifié, saturé en C1-6, notamment méthyle, éthyle, propyle, isopropyle, n-butyle, iso-butyle, tert-butyle, pentyle ou hexyle;

- Ra2 et Ra3, présents sur le même cycle ou chacun sur un cycle différent, représentent, indépendamment l'un de l'autre, un hydrogène, un halogène, ou un groupement de formule -Xa-Ga-Pa (II), sous réserve qu'au moins l'un des radicaux Ra2 et/ou Ra3 représente un groupement de formule (II), dans laquelle :

  - Xa est choisi parmi les groupements -O-, -S-, -SO-, -$SO_2$-, -NH- ou -NR4- avec R4 représentant un radical carboné linéaire, ramifié et/ou cyclique, saturé et/ou insaturé, comprenant 1 à 30 atomes de carbone, éventuellement substitué par un ou plusieurs groupements choisis parmi =O, OH, $NH_2$, et les atomes d'halogènes; et/ou éventuellement interrompu par un ou plusieurs hétéroatomes choisis parmi O, N, P, Si et S;

  - Ga est un radical carboné divalent linéaire, ramifié et/ou cyclique, saturé et/ou insaturé, comprenant 1 à 32 atomes de carbone, éventuellement substitué par un ou plusieurs groupements choisis parmi =O, OH, $NH_2$, et les atomes d'halogènes; et/ou éventuellement interrompu par un ou plusieurs hétéroatomes choisis parmi O, N, P, Si et S;

- Pa est un groupement polymérisable choisi parmi l'une des formules suivantes:

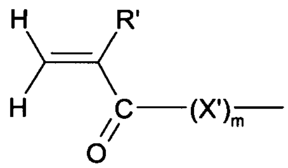

(IIIa)

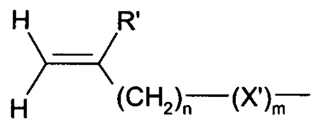

(IIIb)

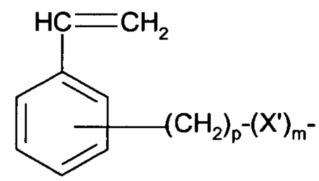

(IIIc)

dans lesquelles :

- R' représente H ou un radical hydrocarboné, linéaire ou ramifié, saturé en C1-6,
- X' représente O, NH ou NR" avec R" représentant un radical choisi parmi les radicaux alkyles en C1-6, aryles en C6-10, aryl(C6-10)alkyles(C1-6) ou alkyle(C1-6)aryles(C6-10), les groupements alkyles et/ou aryles pouvant en outre être substitués par un ou plusieurs groupements choisis parmi OH, halogène, alcoxy en C1-6 et aryloxy en C6-10; et
- m est égal à 0 ou 1;n est égal à 0 ou 1; p est égal à 0, 1 ou 2;

- B représente l'un des groupements aromatiques divalents suivants (IVa) à (IVd) :

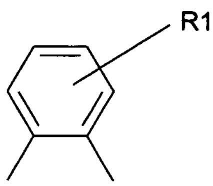

(IVa)

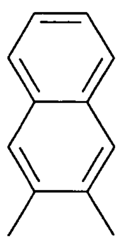

(IVb)

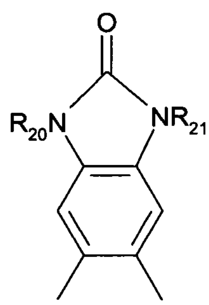

(IVc)

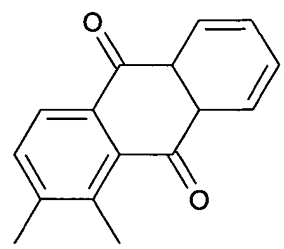

(IVd)

dans lesquels :

- R1 est un radical carboné linéaire, ramifié et/ou cyclique, saturé et/ou insaturé, comprenant 1 à 32 atomes de carbone, éventuellement substitué par un ou plusieurs groupements choisis parmi =O, OH, $NH_2$, et les atomes d'halogènes;
- R20 et R21 sont, indépendamment l'un de l'autre, un atome d'hydrogène, un radical alkyle linéaire ou ramifié en C1-8, un radical cyclopentyle, cyclohexyle, cyclooctyle, cyclodécyle, cyclododécyle, benzyle, naphtyle ou phényle.

De préférence, le ou les monomères à effet optique de formule (I) peuvent être présents en une quantité de 0,01 à 100% en poids, notamment 0,1 à 99,99% en poids, encore mieux de 0,5 à 70% en poids, voire de 1 à 40% en poids, par exemple de 1,5 à 30% en poids, par rapport au poids de la séquence le/les comprenant.
De préférence, le ou les monomères à effet optique de formule (I) peuvent être présents en une quantité de 0,01 à 70% en poids, notamment 0,1 à 50% en poids, encore mieux 0,5 à 30% en poids, voire 1 à 20% en poids, par rapport au poids total du polymère.
Chacune des séquences du polymère selon l'invention peut comprendre au moins un monomère de formule (I), qui peut être identique ou différent selon la séquence.
Les monomères additionnels peuvent être présents en une quantité de 0 à 99,99% en poids, notamment 0,01 à 99,9% en poids, encore mieux de 30 à 99,5% en poids, voire de 60 à 99% en poids, par exemple de 70 à 98,5% en poids, par rapport au poids de la séquence les comprenant et comprenant le/les monomères à effet optique de formule

(I). Ils sont bien évidemment présents à raison de 100% en poids dans la ou les éventuelles séquences ne comprenant pas de monomère de formule (I).
Les monomères additionnels peuvent être présents en une quantité de 30 à 99,99% en poids, notamment 50-99,9% en poids, encore mieux 70-99,5% en poids, voire 80 à 99% en poids, par rapport au poids total du polymère.

D'une manière générale et de préférence, la séquence (ou segment) intermédiaire comprend préférentiellement au moins un monomère m1 constitutif de la première séquence qui peut être choisi parmi les monomères additionnels, et au moins un monomère m2 constitutif de la deuxième séquence choisi parmi les monomères additionnels autres que le monomère m1.

Dans un mode de réalisation particulier, le polymère peut comprendre une première séquence qui comprend 0,5 à 15% en poids, de préférence 1 à 10% en poids de monomère(s) de formule (I) et 85 à 99,5% en poids, notamment 90-99% en poids de monomères additionnels, les % étant donnés par rapport au poids total de ladite séquence. La deuxième séquence comprend préférentiellement 100% en poids de monomères additionnels; la séquence (ou segment) intermédiaire comprend préférentiellement au moins un monomère m1 constitutif de la première séquence choisi parmi les monomères additionnels, et au moins un monomère m2 constitutif de la deuxième séquence choisi parmi les monomères additionnels autres que le monomère m1.

En particulier, dans le polymère selon l'invention, la première séquence peut être choisie parmi :

- a) une séquence ayant une Tg supérieure ou égale à 40°C,
- b) une séquence ayant une Tg inférieure ou égale à 20°C,
- c) une séquence ayant une Tg comprise entre 20 et 40°C,

et la deuxième séquence peut être choisie dans une catégorie a), b) ou c) différente de la première séquence.

La séquence comprenant le/les monomère(s) à effet optique de formule (I) présente de préférence une Tg supérieure ou égale à 40°C, notamment supérieure ou égale à 60°C.

Dans ce cas, la ou les autres séquences, si elles ne comprennent pas de monomères à effet optique de formule (I), peuvent présenter une Tg inférieure ou égale à 40°C, notamment inférieure ou égale à 20°C.

Si une deuxième séquence comprend au moins un monomère à effet optique de formule (I), elle peut présenter de préférence une Tg inférieure ou égale à 40°C, notamment inférieur ou égale à 20°C.

Lorsque le polymère comprend une séquence ayant une Tg supérieure ou égale à 40°C, cette séquence peut par exemple avoir une Tg allant de 40 à 150°C, de préférence de 50°C à 120°C, et mieux de 60°C à 120°C.
Elle peut alors notamment comprendre, en totalité ou en partie, des monomères dont les homopolymères ont une Tg dans la gamme souhaitée, notamment supérieure ou égale à 40°C. Elle peut également comprendre des monomères ayant une Tg en dehors de cette gamme. Ces monomères et leur concentration sont choisis de manière adéquate par l'homme du métier, notamment sur la base de la loi de Fox, pour obtenir une séquence de Tg souhaitée.
Parmi ces monomères, on peut citer :

- les méthacrylates de formule : $CH_2 = C(CH_3)\text{-}COOR_1$
  dans laquelle $R_1$ représente un groupe alkyle non substitué, linéaire ou ramifié, contenant de 1 à 4 atomes de carbone, tel qu'un groupe méthyle, éthyle, propyle ou isobutyle, ou bien $R_1$ représente un groupe cycloalkyle $C_4$ à $C_{12}$, notamment isobornyle;
- les acrylates de formule : $CH_2 = CH\text{-}COOR_2$
  dans laquelle $R_2$ représente un groupe tertio butyle ou un groupe cycloalkyle en $C_4$ à $C_{12}$ tel que un groupe isobornyle;
- les (méth)acrylamides de formule : $CH_2 = CR'\text{-}CO\text{-}NR^7R^8$
  avec R' représentant H ou $CH_3$, et $R_7$ et $R_8$, identiques ou différents, représentent un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle; ou encore $R_7$ représente H et $R_8$ représente un groupement 1,1-diméthyl-3-oxobutyl,
- et leurs mélanges.

Parmi les monomères dont les homopolymères ont une température de transition vitreuse Tg supérieure ou égale à 40°C plus particulièrement préférés, on peut citer le méthacrylate de méthyle, le méthacrylate d'éthyle, le méthacrylate d'isobutyle, le (méth)acrylate de tertio-butyle, l'acide (méth)acrylique, le (méth)acrylate d'isobornyle, le N-butylacrylamide, le N-t-butylacrylamide, le N-isopropylacrylamide, le N,N-diméthylacrylamide, le N,N-dibutylacrylamide et leurs mélanges.

Lorsque le polymère comprend une séquence ayant une Tg inférieure ou égale à 20°C, cette séquence peut avoir par exemple une Tg allant de -100 à 20°C, de préférence de -80°C à 15°C et mieux de -50°C à 0°C.
Elle peut alors notamment comprendre, en totalité ou en partie, des monomères dont les homopolymères ont une Tg dans la gamme souhaitée, notamment inférieure ou égale à 20°C. Elle peut également comprendre des monomères

ayant une Tg en dehors de cette gamme. Ces monomères et leur concentration sont choisis de manière adéquate par l'homme du métier, notamment sur la base de la loi de Fox, pour obtenir une séquence de Tg souhaitée.
Parmi ces monomères, on peut citer :

- les acrylates de formule $CH_2 = CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,
- les méthacrylates de formule $CH_2 = C(CH_3)\text{-}COOR_4$,
  $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5\text{-CO-O-CH} = CH_2$
  où $R_5$ représente un groupe alkyle en $C_4$ à $C_{12}$ linéaire ou ramifié ;
- les éthers de vinyle et d'alkyles en $C_4$-$C_{12}$, notamment l'éther de vinyle et de méthyle, et l'éther de vinyle et d'éthyle;
- les N-(alkyl en $C_4$ à $C_{12}$)acrylamides, tels que le N-octylacrylamide,
- et leurs mélanges.

Parmi ces monomères, on peut citer plus particulièrement l'acrylate de méthyle, l'acrylate d'éthyle, l'acrylate d'isobutyle, le (méth)acrylate d'éthyl-2-hexyle et leurs mélanges.
Lorsque le polymère comprend une séquence ayant une Tg comprise entre 20°C et 40°C, elle peut notamment comprendre, en totalité ou en partie, des monomères dont les homopolymères ont une Tg dans la gamme souhaitée, et parmi lesquels on peut citer le méthacrylate de n-butyle, l'acrylate de cyclohexyle, l'acrylate de cyclododécyle, l'acrylate de néopentyle, l'isodécylacrylamide et leurs mélanges.
Dans un mode de réalisation préféré, le polymère selon l'invention comprend dans au moins une séquence, préférentiellement dans chacune des séquences, au moins un monomère choisi parmi les esters d'acide (méth)acrylique; éventuellement, il peut comprendre en outre au moins un deuxième monomère choisi parmi l'acide acrylique et l'acide méthacrylique, et leurs mélanges.
Préférentiellement, tous les monomères autres que les monomères à effets optiques de formule (I) sont choisis parmi les esters d'acide (méth)acrylique et l'acide (méth)acrylique.
Selon un premier mode de réalisation, le polymère selon l'invention comprend au moins une première séquence ayant une Tg supérieure ou égale à 40°C, de préférence supérieure ou égale à 50°C, notamment supérieure ou égale à 60°C, et au moins une deuxième séquence ayant une Tg inférieure ou égale à 20°C, de préférence inférieure ou égale à 10°C, notamment inférieure ou égale à 0°C.
De préférence, la proportion, dans le polymère final, de la séquence ayant une Tg supérieure ou égale à 40°C peut être de 20 à 95% en poids par rapport au poids du polymère final, mieux de 30 à 80% et encore mieux de 50 à 75%.
De préférence, la proportion, dans le polymère final, de la séquence ayant une Tg inférieure ou égale à 20°C peut être de 5 à 80% en poids par rapport au poids du polymère final, de préférence de 15 à 50% et mieux de 25 à 45%.
La masse moyenne en poids (Mw) du polymère selon l'invention est de préférence inférieure ou égale à 300 000; elle va par exemple de 35 000 à 200 000, et mieux de 40000 à 150 000.
La masse moyenne en nombre (Mn) du polymère selon l'invention est de préférence inférieure ou égale à 70 000, elle va par exemple de 5 000 à 60 000, et mieux de 6000 à 50 000.
On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique et UV).
De préférence, l'indice de polydispersité du polymère selon l'invention est supérieur à 2, par exemple allant de 2 à 9, de préférence supérieur ou égal à 2,5, par exemple allant de 2,5 à 8, et mieux supérieur ou égal à 2,8 et notamment, allant de 2,8 à 7. L'indice de polydispersité Ip du polymère est égal au rapport de la masse moyenne en poids Mw sur la masse moyenne en nombre Mn.
Le polymère selon l'invention présente de préférence une longueur d'onde d'absorption comprise entre 200 et 550 nm, notamment entre 220 et 500 nm, voire entre 240 et 490 nm.
Il présente de préférence une longueur d'onde d'émission comprise entre 350 et 700 nm, notamment entre 390 et 650 nm, voire entre 400 et 600 nm.
Le polymère selon l'invention peut être obtenu par polymérisation radicalaire en solution selon le procédé de préparation suivant :

- on peut introduire une partie du solvant de polymérisation dans un réacteur adapté, on chauffe jusqu'à atteindre la température adéquate pour la polymérisation (typiquement entre 60 et 120°C),
- une fois cette température atteinte, on peut ajouter les monomères constitutifs de la première séquence, en présence d'une partie de l'initiateur de polymérisation,

- au bout d'un temps T correspondant à un taux de conversion maximum de 90% de préférence, on peut introduire les monomères constitutifs de la deuxième séquence et l'autre partie de l'initiateur,
- on laisse réagir le mélange pendant un temps T' (allant notamment de 3 à 6 heures) au bout duquel le mélange est ramené à température ambiante (25°C), de manière à obtenir le polymère en solution dans le solvant de polymérisation.

Par solvant de polymérisation, on entend un solvant, ou un mélange de solvants, notamment choisi parmi l'acétate d'éthyle, l'acétate de butyle, les alcools en C1-C6 tels que l'isopropanol, l'éthanol, les alcanes aliphatiques tels que l'isododécane et leurs mélanges. De préférence, le solvant de polymérisation est un mélange d'acétate de butyle et d'isopropanol ou est l'isododécane.

De préférence, le polymère selon l'invention n'est pas hydrosoluble, c'est à dire que le polymère n'est pas soluble dans l'eau ou dans un mélange d'eau et de monoalcools linéaires ou ramifiés ayant de 2 à 5 atomes de carbone comme l'éthanol, l'isopropanol ou le n-propanol, sans modification de pH, à une teneur en matière active d'au moins 1 % en poids, à température ambiante (25°C).

L'invention concerne également des compositions notamment cosmétiques comprenant au moins un polymère de structure spécifique, tel qu'il a été décrit ci-dessus, dans un milieu physiologiquement, notamment cosmétiquement, acceptable.

Les polymères selon l'invention peuvent être présents, seuls ou en mélange, dans les compositions selon l'invention en une quantité de 0,01 à 75% en poids, de préférence 0,1 à 70% en poids, notamment 1 à 65% en poids, voire 3 à 60% en poids, et encore mieux 5 à 50% en poids, préférentiellement 6 à 25% en poids, par rapport au poids total de la composition.

Ils peuvent être présents dans la composition sous forme solubilisée, par exemple dans l'eau, dans une huile ou dans un solvant organique, ou bien sous forme de dispersion aqueuse ou organique.
Avantageusement, les polymères selon l'invention sont solubles ou dispersibles dans au moins une des phases de la composition qui les comprend.

Les compositions cosmétiques ou pharmaceutiques selon l'invention comprennent, outre lesdits polymères, un milieu physiologiquement acceptable, notamment cosmétiquement, dermatologiquement ou pharmaceutiquement, acceptable, c'est-à-dire un milieu compatible avec les matières kératiniques telles que la peau du visage ou du corps, les cheveux, les cils, les sourcils et les ongles.

La composition peut ainsi comprendre, un milieu hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le pentylène glycol, et les polyéthylène glycols, ou bien encore des éthers en $C_2$ et des aldéhydes en $C_2$-$C_4$ hydrophiles.
L'eau ou le mélange d'eau et de solvants organiques hydrophiles peut être présent dans la composition selon l'invention en une teneur allant de 0, 1 % à 99% en poids, par rapport au poids total de la composition, et de préférence de 10% à 80% en poids.
La composition peut également être anhydre.

La composition peut également comprendre une phase grasse qui peut comprendre des corps gras liquides à température ambiante (25°C en général) et/ou des corps gras solides à température ambiante tels que les cires, les corps gras pâteux, les gommes et leurs mélanges. Ces corps gras peuvent être d'origine animale, végétale, minérale ou synthétique. Cette phase grasse peut, en outre, contenir des solvants organiques lipophiles.
Comme corps gras liquides à température ambiante, appelés souvent huiles, utilisables dans l'invention, on peut citer : les huiles hydrocarbonées d'origine animale telles que le perhydrosqualène; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement

un groupement phényle, comme les phényl triméthicones, les phényltriméthylsiloxydiphényl siloxanes, les diphénylméthyldiméthyltrisiloxanes, les diphényl diméthicones, les phényl diméthicones, les polyméthylphényl siloxanes ; leurs mélanges.

Ces huiles peuvent être présentes en une teneur allant de 0,01 à 90%, et mieux de 0,1 à 85% en poids, par rapport au poids total de la composition.

La composition selon l'invention peut également comprendre un ou plusieurs solvants organiques, physiologiquement acceptables.

Ces solvants peuvent être généralement présents en une teneur allant de 0,1 à 90%, de préférence de 0,5 à 85%, de préférence encore de 10 à 80% en poids, par rapport au poids total de la composition, et mieux de 30 à 50 %.

On peut notamment citer, outre les solvants organiques hydrophiles cités plus haut, les cétones liquides à température ambiante tels que méthyléthylcétone, méthylisobutylcétone, diisobutylcétone, l'isophorone, la cyclohexanone, l'acétone ; les éthers de propylène glycol liquides à température ambiante tels que le monométhyléther de propylène glycol, l'acétate de monométhyl éther de propylène glycol, le mono n-butyl éther de dipropylène glycol ; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à 25°C tels que le diéthyléther, le diméthyléther ou le dichlorodiéthyléther ; les alcanes liquides à 25°C tels que le décane, l'heptane, le dodécane, l'isododécane, le cyclohexane; les composés cycliques aromatiques liquides à 25°C tels que le toluène et le xylène ; les aldéhydes liquides à 25°C tels que le benzaldéhyde, l'acétaldéhyde et leurs mélanges.

Par cire au sens de la présente invention, on entend un composé lipophile, solide à température ambiante (25°C), à changement d'état solide/liquide réversible, ayant un point de fusion supérieur ou égal à 25°C pouvant aller jusqu'à 120°C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent un point de fusion supérieur à 30°C et mieux supérieur à 45°C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxydiméthicone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0,1 à 50% en poids de cires, par rapport au poids total de la composition et mieux de 1 à 30% en poids.

La composition selon l'invention peut en outre comprendre, dans une phase particulaire, des pigments et/ou des nacres et/ou des charges habituellement utilisés dans les compositions cosmétiques.

La composition peut également comprendre d'autres matières colorantes choisies parmi les colorants hydrosolubles et/ou les colorants liposolubles bien connus de l'homme du métier.

Par pigments, il faut comprendre des particules de toute forme, blanches ou colorées, minérales ou organiques, insolubles dans le milieu physiologique, destinées à colorer la composition.

Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.

Par nacres, il faut comprendre des particules de toute forme irisées, notamment produites par certains mollusques dans leur coquille ou bien synthétisées.

Les pigments peuvent être présents dans la composition à raison de 0,01 à 25% en poids de la composition finale, et de préférence à raison de 3 à 10% en poids. Ils peuvent être blancs ou colorés, minéraux ou organiques,. On peut citer les oxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium. On peut encore citer les pigments D&C et les laques couramment employées pour conférer aux lèvres et à la peau un effet de maquillage, qui sont des sels de calcium, de baryum, d'aluminium, de strontium ou de zirconium.

Les nacres peuvent être présentes dans la composition à raison de 0,01 à 20% en poids, de préférence à un taux de l'ordre de 3 à 10% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Parmi les colorants, liposolubles ou hydrosolubles, qui peuvent être présents dans la composition, seul ou en mélange, à raison de 0,001 à 15% en poids, de préférence 0,01 à 5% en poids et notamment de 0,1 à 2% en poids, par rapport au poids total de la composition, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le bleu de méthylène, le carmin de cochenille, les colorants halogéno-acides, azoïques, anthraquinoniques, le sulfate de cuivre ou de fer, le brun Soudan, le rouge Soudan et le rocou, ainsi que le jus de betterave et le carotène.
La composition selon l'invention peut comprendre en outre en outre une ou plusieurs charges, notamment en une teneur allant de 0,01% à 50% en poids, par rapport au poids total de la composition, de préférence allant de 0,02% à 30% en poids. Les charges peuvent être minérales ou organiques de toute forme, plaquettaires, sphériques ou oblongues. On peut citer le talc, le mica, la silice, le kaolin, les poudres de polyamide (Nylon®), de poly-β-alanine et de polyéthylène, les poudres de polymères de tétrafluoroéthylène (Téflon®), la lauroyllysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition selon l'invention peut également comprendre des ingrédients couramment utilisés en cosmétique, tels que les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, ou leurs mélanges.
Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter sous la forme d'une suspension, une dispersion notamment d'huile dans de l'eau grâce à des vésicules; une solution aqueuse ou huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; une poudre libre, compacte ou coulée; une pâte anhydre. Cette composition peut avoir l'aspect d'une lotion, d'une crème, d'une pommade, d'une pâte souple, d'un onguent, d'une mousse, d'un solide coulé ou moulé et notamment en stick ou en coupelle, ou encore de solide compacté.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition cosmétique selon l'invention peut se présenter sous la forme d'un produit de soin et/ou de maquillage de la peau du corps ou du visage, des lèvres, des ongles, des cils, des sourcils et/ou des cheveux, d'un produit solaire ou autobronzant, d'un produit capillaire pour le soin, le traitement, la mise en forme, le maquillage ou la coloration des cheveux.

Elle peut ainsi se présenter sous la forme d'une composition de maquillage, notamment un produit pour le teint tel qu'un fond de teint, un fard à joues ou à paupières; un produit pour les lèvres tel qu'un rouge à lèvres ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux).
Elle peut également se présenter sous forme d'une composition de protection ou de soin de la peau du visage, du cou, des mains ou du corps, notamment une composition anti-rides, une composition hydratante ou traitante; une composition anti-solaire ou de bronzage artificiel.
Elle peut encore se présenter sous forme d'un produit capillaire, notamment pour la coloration, le maintien de la coiffure, la mise en forme des cheveux, le soin, le traitement ou le nettoyage des cheveux, telle que des shampooings, des

gels, des lotions de mise en plis, des lotions pour le brushing, des compositions de fixation et de coiffage telles que les laques ou spray.

Dans un premier mode de réalisation particulier de l'invention, la composition peut se présenter sous la forme d'un vernis à ongles qui peut comprendre, outre le ou les polymères selon l'invention, un ou plusieurs solvants organiques, au moins un polymère filmogène et éventuellement au moins un pigment et/ou au moins un colorant.

Dans un second mode de réalisation particulier de l'invention, la composition peut se présenter sous la forme d'un fond de teint qui peut comprendre, outre le ou les polymères selon l'invention, au moins une huile dans une phase grasse, au moins un pigment et éventuellement une phase aqueuse.

Dans un troisième mode de réalisation de l'invention, la composition peut se présenter sous la forme d'une composition anti-age ou anti-rides, destinée de préférence à être appliquée sur le visage et/ou le cou, en particulier sur les zones ridées du visage, et notamment autour des yeux.

En effet, on a constaté de façon surprenante que l'utilisation de polymères selon l'invention permettait l'obtention d'une composition susceptible d'être appliquée sur la peau et donnant un effet tenseur immédiat des rides et/ou des ridules déjà formées; l'utilisation de ces polymères en tant qu'agents tenseurs est particulièrement avantageuse, car ils permettent de former un film tenseur efficace présentant une rigidité efficace tout en étant souple afin d'éviter un tiraillement gênant des matières kératiniques telles que la peau, lors de l'application d'une composition comprenant de tels agents.

Dans ce cas, le polymère séquencé est de préférence non élastomère et non hydrosoluble. Par polymère non hydrosoluble, on entend que le polymère n'est pas soluble dans l'eau ou dans un mélange d'eau et de monoalcools linéaires ou ramifiés en C2-C5, comme l'éthanol, l'isopropanol ou le n-propanol, sans modification de pH, à une teneur en matière active d'au moins 1% en poids, à température ambiante (25°C).

Dans ce 3$^{ème}$ mode de réalisation, la première séquence a avantageusement une Tg supérieure ou égale à 85°C, par exemple comprise entre 90 et 150°C, de préférence comprise entre 100°C et 120°C. De préférence, la proportion de la séquence de Tg supérieure ou égale à 85°C est de 50 à 90% en poids du poids du polymère final, et de préférence de 60 à 80% en poids du polymère final. La deuxième séquence a avantageusement une Tg inférieure ou égale à 20°C, par exemple comprise entre -100°C et 20°C, de préférence comprise entre -80°C et 15°C et mieux comprise entre -70°C et 10°C. De préférence, la proportion de la séquence de Tg inférieure ou égale à 20°C est de 5 à 50% en poids de polymère final et de préférence de 10 à 40% en poids du poids du polymère final.

La composition selon l'invention peut également comprendre au moins un actif anti-âge notamment choisi parmi les agents desquamants, les agents hydratants, les agents stimulant la prolifération et/ou la différenciation des kératinocytes, les agents stimulant la synthèse du collagène et/ou de l'élastine ou prévenant leur dégradation, les agents dépigmentants, les agents anti-glycation, les agents stimulant la synthèse de glycoaminoglycannes, les agents dermo-décontractants ou myorelaxants, les agents anti-oxydants et anti-radicalaires, et leurs mélanges.

L'invention a encore pour objet l'utilisation des polymères selon l'invention en tant qu'agent tenseur dans une composition cosmétique, notamment dans une composition antirides.

Un autre objet de la présente invention est un procédé de traitement cosmétique d'une peau ridée, tel que le contour de l'oeil, comprenant une étape consistant à appliquer sur ladite peau une composition cosmétique comprenant, dans un milieu cosmétiquement acceptable, au moins un polymère tel que défini précédemment.

L'invention a également pour objet un procédé de traitement cosmétique, notamment de maquillage ou de soin des matières kératiniques, telles que la peau du corps ou du visage, des lèvres, des ongles, des cils, des sourcils et/ou des cheveux, comprenant l'application sur lesdites matières d'une composition cosmétique telle que définie précédemment.

L'invention est illustrée plus en détail dans les exemples suivants.

<u>Méthode de mesure de la longueur d'ondes (émission et absorption)</u>

La mesure des longueurs d'ondes est réalisée à l'aide d'un fluorimètre Varian Cary Eclipse.

Sauf indication contraire, cette mesure est effectuée de la manière suivante :

> On dispose 20 mg de produit dans un cylindre de 50 ml. Afin de solubiliser le produit, on complète ledit cylindre jusqu'à 50 ml, à l'aide d'un solvant approprié, par exemple le dichlorométhane (DCM), le chloroforme, l'isododécane, l'heptane ou le diméthylsulfoxyde (DMSO). La solution résultante est mélangée et on en prélève 250 microlitres que l'on dispose dans un cylindre de 50 ml, puis que l'on complète à nouveau avec le solvant jusqu'à 50 ml. On mélange le tout et l'on prélève un échantillon de la solution que l'on dispose dans une cuve fermée, en quartz et d'épaisseur 10 mm, qui est alors placée dans la chambre de mesure.

**Exemple 1**

1/ première étape

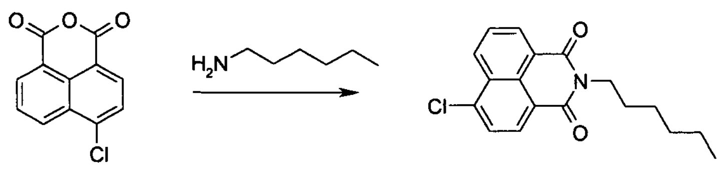

Dans un ballon de 2 litres, sous atmosphère inerte (azote), on dispose 26,9 g (0,116 mol) de 4-chloro-1,8-naphthalic anhydride, puis l'on ajoute 450 ml de toluène. On mélange à 500 tr/min. pendant quelques minutes, puis on introduit goutte-à-goutte 14,0 g (0,139 mol) de 1-hexylamine préalablement mis en solution dans 100 ml de toluène. Le mélange est chauffé au reflux puis l'on ajoute encore 50 ml de toluène. Le chauffage au reflux est poursuivi pendant 24 heures. Le mélange réactionnel résultant est alors laissé à refroidir à température ambiante. On concentre le produit sous pression réduite. On récupère la phase organique et on la recristallise dans l'éthanol. On obtient 29,0 g de cristaux jaune pâle (rendement 79,4%)

Caractérisation

$^1$H-NMR ($CDCl_3$, 400MHz) δ : 8.67-8.65 (2H), 8.51-8.49 (1H), 7.87-7.85 (1H), 7.83-7.81 (1 H), 4.18-4.14 (2H), 1.74-1.70 (2H), 1.44-1.32 (6H), 0,90-0.87 (3H).

2/ deuxième étape

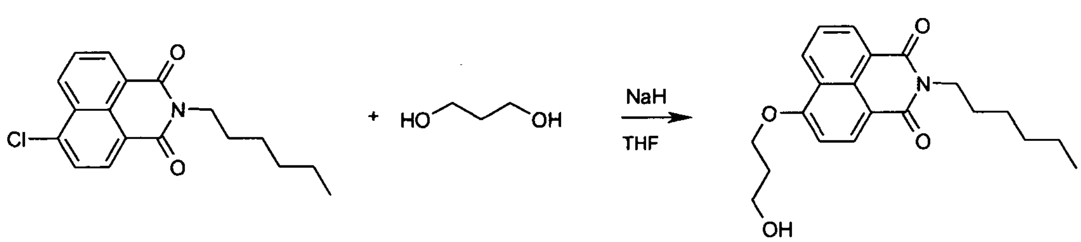


On dispose 1,5 g (64 mmol) d'hydrate de sodium (NaH) dans un ballon sous atmosphère inerte d'argon; on ajoute 300 ml de THF (tetrahydrofurane) et on refroidit le mélange réactionnel à 0°C. On ajoute 2,4 g (32 mmol) de 1,3-propanediol préalablement mélangé avec 100 ml de THF, et l'on mélange vigoureusement. On chauffe le mélange à 60°C pendant 20 minutes puis on le refroidit jusqu'à température ambiante (25°C). On ajoute alors un mélange constitué de 10 g (32 mmol) de naphthalimide dans 150 ml de THF. Le mélange est chauffé au reflux pendant 3 heures, puis on ajoute 10 ml d'éthanol, on chauffe pendant 15 minutes, et l'on évapore le solvant sous pression réduite afin d'obtenir un résidu verdâtre qui est lavé à l'acétate d'éthyle et à l'eau. On obtient alors 8,6 g d'une poudre jaune pâle (rendement 75,5%).

Caractérisation

$^1$H-NMR ($CDCl_3$, 400MHz) δ: 8.61-8.59 (2H), 8.55-8.53 (1H), 7.72-7.68 (1H), 7.08-7.06 (1H), 4.45-4.42 (2H), 4.17-4.13 (2H), 4.00-3.97 (2H), 2.28-2.22 (2H), 1.76-1.70 (2H), 1.44-1.32 (6H), 0.90-0.87 (3H).

3/ Troisième étape

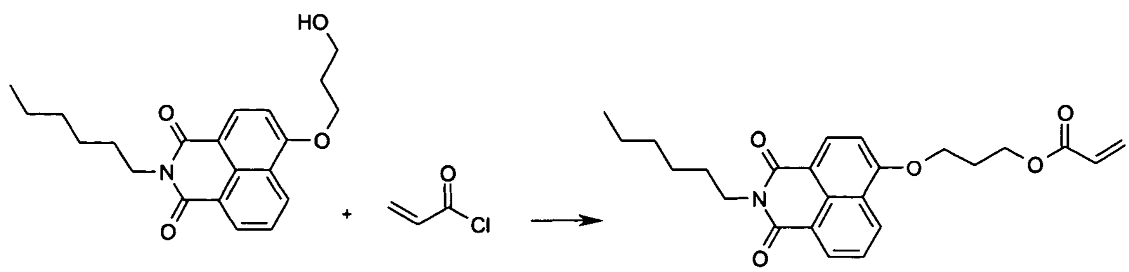

On dispose 8,0 g (22,5 mmol) de 4-(oxy-3-propanol)-N-hexyl naphthalimide dans un ballon tricol équipé d'un réfrigérant et placé sous atmosphère inerte d'argon. On ajoute 150 ml de dichlorométhane et la solution est agitée jusqu'à obtention d'une solution homogène. On ajoute alors 4,6 g (45,0 mmol) de triéthanolamine puis 4,1 g (45,0 mmol) de chlorure d'acryloyle, sous agitation à 0°C. On suit l'évolution de la réaction par CCM (chromatographie couche mince), et lorsqu'il n'y a plus de composés de départ, on ajoute 50 ml d'eau. La solution réactionnelle est alors lavée avec une solution saturée en NaCl puis séchée sur sulfate de sodium. On évapore les solvants sous pression réduite et l'on obtient 9,2 g d'une poudre jaune pâle (rendement 100%).

Caractérisation

$^{1}$H-NMR ($CDCl_3$, 400MHz) δ: 8.59-8.57 (1H), 8.54-8.52 (1H), 8.51-8.50 (1H), 7.70-7.66 (1H), 7.03-7.01 (1H), 6.44-6.40 (1H), 6.17-6.10 (1H), 5.86-5.83 (1H), 4.49-4.46 (2H), 4.39-4.36 (2H), 4.16-4.12 (2H), 2.38-2.35 (2H), 1.71-1.69 (2H), 1.44-1.31 (6H), 0.89-0.86 (3H).

## Exemple 2

On introduit 33 g d'isododécane dans un réacteur de 500 ml, puis on chauffe jusqu'à 90°C.
On dissout 2 g de monomère azurant préparé selon l'exemple 1 dans 10 ml de toluène puis on ajoute 40 g d'acrylate d'isobornyle, 28 g de méthacrylate d'isobutyle, 37 g d'isododécane et 0,6 g d'amorceur 2,5-bis(2-éthylhexanoylperoxy)-2,5-diméthylhexane (Trigonox® 141 d'Akzo Nobel). Ce mélange est ajouté en 1 heure dans le réacteur à 90°C. On maintient le tout à 90°C pendant 1 h 30.
On introduit ensuite au mélange précédent, à 90°C et en 30 minutes, 30 g d'acrylate d'éthyle-2-hexyle, 30 g d'isododécane et 0,4 g de 2,5-bis(2-éthylhexanoylperoxy)-2,5-diméthylhexane. Le mélange est maintenu 4 heures à 90°C, puis l'ensemble est refroidi à 25°C.
Après remplacement du toluène par de l'isododécane, on obtient une solution à 50% de matière sèche de polymère dans l'isododécane.
Le polymère présente une masse moyenne en poids (Mw) de 48 500 et une masse moyenne en nombre (Mn) de 12 300, soit un indice de polydispersité Ip de 3.94.
Ce polymère comprend une première séquence acrylate d'isobornyle, méthacrylate d'isobutyle, monomère azurant selon l'invention, et une seconde séquence acrylate d'éthyle-2-hexyle, ainsi qu'un segment intermédiaire.

**Exemple 3**

1/première étape

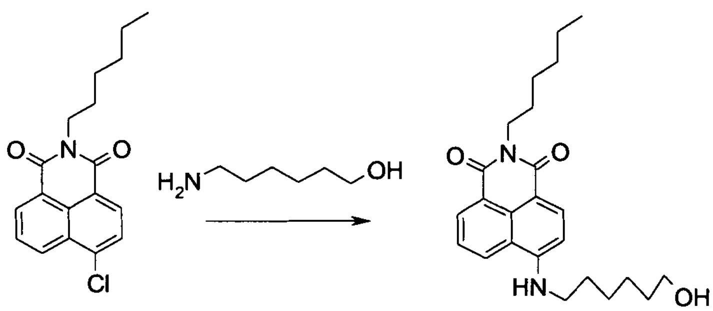

Dans un ballon, sous argon, on mélange dans 170 ml d'éthanol, 2,71 g (10,85 mmol) de $CuSO_4$, $5H_2O$ et 10,07 g (31,9 mmol) de 4-chloro-N-hexyl-1,8-napthalimide, puis le milieu réactionnel est porté à reflux.
On coule 7,7 g (65,7 mmol) d'aminohexane-6-ol sur le milieu réactionnel goutte-à-goutte puis on maintient l'agitation pendant 16 heures. On refroidit ensuite la solution jusqu'à 25°C et le produit est précipité par ajout de 200 ml d'eau, puis filtré sur fritté. On obtient un produit solide vert-jaune qui est repris dans 100 ml de dichlorométhane. On lave à l'eau puis on évapore la phase aqueuse pour obtenir une huile que l'on filtre sur silice (0,043-0,060 microns, élution au dichlorométhane puis à l'acétone).
On obtient au final 1,6 g d'une huile jaune (rendement 12,6%).

Caractérisation

$^1H$-NMR ($CDCl_3$, 400MHz) $\delta$ ppm: 0,87 (t, 3H, $CH_3$); 1,50 (m, 8H, $CH_2$); 1,81 (m, 2H, $NCH_2CH_2$); 3,40 (t, 2H, $CH_2OH$); 3,68 (t, 2H, $NHCH_2$); 4,14 (t, 2H, $NCH_2$); 5,26 (s, 1 H, NH); 6,71 (d, 1 H, ArH); 7,60 (t, 1 H, ArH); 8,06 (d, 1 H, ArH); 8,43 (d, 1 H, ArH); 8,57 (d, 1 H, ArH).

2/ deuxième étape

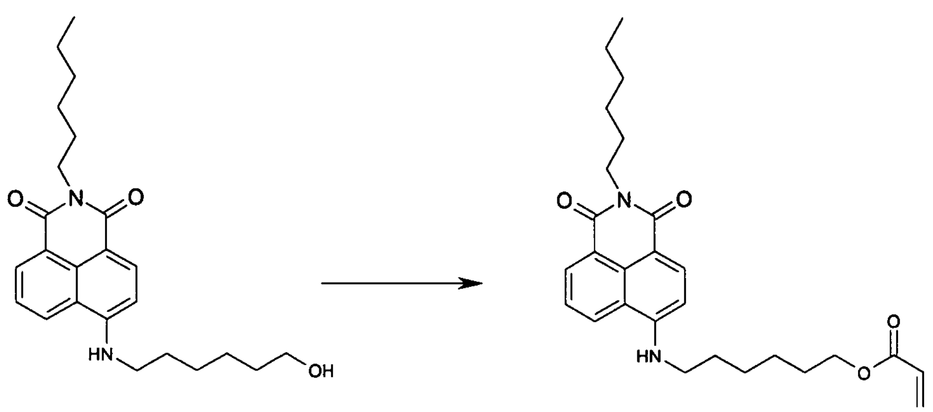

On dispose 1,5 g (3,9 mmol) de 4-(amino-6-hexanol)-N-hexyl naphthalimide dans un ballon tricol équipé d'un réfrigérant et placé sous atmosphère inerte d'argon. On ajoute 50 ml de dichlorométhane et la solution est agitée jusqu'à obtention d'une solution homogène. On ajoute alors 0,8 g (7,9 mmol) de triéthanolamine puis 0,7 g (7,9 mmol) de chlorure d'acryloyle, sous agitation à 0°C. Puis on laisse la température s'élever jusqu'à 25°C. On suit l'évolution de la réaction par CCM et lorsque l'on constate qu'il n'y a plus de produits de départ, on ajoute 10 ml d'eau. La solution

réactionnelle est alors lavée avec une solution saturée en NaCl puis séchée sur sulfate de sodium. On évapore les solvants sous pression réduite et l'on obtient 1,7 g (rendement 99%) d'une poudre orange.

Caractérisation

$^1$H-NMR (400 MHz ; $CDCl_3$) δ ppm : 0.87 (t, 3H, $CH_3$); 1.50 (m, 8H, $CH_2$); 1.81 (m, 2H, $NCH_2CH_2$); 3.68 (t, 2H, $NHCH_2$); 4.17 (m, 4H, $NCH_2$; $CH_2O$); 5.26 (s, 1 H, NH); 5.77 (d, 1 H C=CH) ; 6.10 (m, 1 H C=CH) ; 6.39 (d, 1 H, CH=C) ; 6.70 (d, 1 H, ArH); 7.60 (t, 1 H, ArH) ; 8.06 (d, 1 H, ArH); 8.43 (d, 1 H, ArH) ; 8.57 (d, 1 H, ArH).

**Exemple 4**

On introduit 50 g d'acétate d'éthyle dans un réacteur de 500 ml, puis on chauffe à 78°C en 1 heure. On ajoute ensuite, à 78°C et en 40 minutes, 25 g de méthacrylate de méthyle, 5 g d'acide acrylique, 5 g de monomère fluorescent préparé à l'exemple 3 en solution dans 20 g d'éthanol, et 0,3 g de 2,5-bis(2-éthylhexanoylperoxy)-2,5-diméthylhexane (Trigonox®141 d'Akzo Nobel). On maintient le mélange pendant 1 heure à 78°C. On introduit ensuite, à 78°C et en 40 minutes, 15 g d'acrylate de méthyle et 0,2 g de 2,5-bis(2-éthylhexanoylperoxy)-2,5-diméthylhexane. Le mélange est maintenu 3 heures à 78°C, puis dilué par 75 g d'acétate de butyle. On distille l'acétate d'éthyle et l'éthanol sous pression réduite, puis on ajoute 50 g d'acétate de butyle et l'on distille 50 g d'acétate de butyle.
On obtient une solution à 40% de matière sèche de polymère dans l'acétate de butyle.
Ce polymère comprend une première séquence méthacrylate de méthyle, acide acrylique et monomère selon l'invention, et une seconde séquence acrylate de méthyle, ainsi qu'un segment intermédiaire.

**Exemple 5**

On prépare un fond de teint anhydre comprenant (% en poids) :

- cire de polyéthylène 12%
- huiles siliconées volatiles 25%
- phényltriméthicone 20%
- Microsphères de polyméthylméthacrylate 12%
- Polymère de l'exemple 2 6%
- Isododécane qsp 100%

Préparation :

On fait fondre les cires puis, quand tout est limpide, on ajoute la phényl triméthicone sous agitation, et les huiles de silicones; on ajoute ensuite les microsphères, l'isododécane et le polymère. On homogénéise pendant 15 minutes puis on coule la composition résultante que l'on laisse refroidir.
On obtient un fond de teint anhydre.

**Exemple 6**

On prépare un vernis à ongles comprenant :

- 20% en poids de polymère selon l'exemple 4
- qsp 100% solvants organiques (acétate de butyle et acétate d'éthyle).

**Exemple 7**

On prépare un stick de rouge à lèvres comprenant :

- Cire de polyéthylène 15 %
- Polymère de l'exemple 2 10 % en MA
- Polyisobutène hydrogéné (Parléam de Nippon Oil Fats) 25 %
- Pigments 10 %
- Isododécane qsp 100%

**Exemple 8**

On introduit 100 g d'isododécane dans un réacteur de 1 litre, puis on chauffe jusqu'à 90°C.

On dissout 18 g de monomère azurant préparé selon l'exemple 1 dans 90 ml de toluène puis on ajoute 96 g d'acrylate d'isobornyle, 96 g de méthacrylate d'isobornyle, 110 g d'isododécane et 1,8 g d'amorceur 2,5-bis(2-éthylhexanoylperoxy)-2,5-diméthylhexane (Trigonox® 141 d'Akzo Nobel). Ce mélange est ajouté en 1 heure dans le réacteur à 90°C. On maintient le tout à 90°C pendant 1 h 30.
On introduit ensuite au mélange précédent, à 90°C et en 30 minutes, 90 g d'acrylate d'éthyle-2-hexyle, 90 g d'isododécane et 1,2 g de 2,5-bis(2-éthylhexanoylperoxy)-2,5-diméthylhexane. Le mélange est maintenu 3 heures à 90°C, puis l'ensemble est refroidi à 25°C.
Après remplacement du toluène par de l'isododécane, on obtient une solution à 46% de matière sèche de polymère dans l'isododécane.
Le polymère présente une masse moyenne en poids (Mw) de 65100 et une masse moyenne en nombre (Mn) de 16000, soit un indice de polydispersité Ip de 4,07.
Ce polymère comprend une première séquence acrylate d'isobornyle, méthacrylate d'isobornyle, monomère azurant selon l'invention, et une seconde séquence acrylate d'éthyle-2-hexyle, ainsi qu'un segment intermédiaire.

**Revendications**

1. Polymère séquencé comprenant au moins une première séquence et au moins une deuxième séquence incompatibles l'une avec l'autre, lesdites première et deuxième séquences étant reliées entre elles par un segment intermédiaire comprenant au moins un monomère constitutif de la première séquence et au moins un monomère constitutif de la deuxième séquence, **caractérisé en ce qu'**au moins une des séquences comprend au moins un monomère de formule (I) :

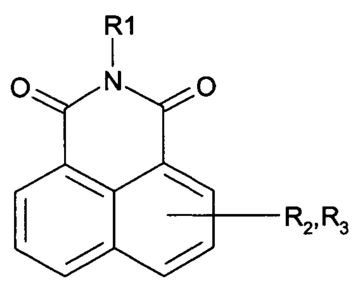

(I)

dans laquelle :

- R1 représente un radical carboné linéaire, ramifié et/ou cyclique, saturé et/ou insaturé, comprenant 1 à 32 atomes de carbone; éventuellement substitué par un ou plusieurs groupements choisis parmi =O, OH, $NH_2$, et les atomes d'halogènes; et/ou éventuellement interrompu par un ou plusieurs hétéroatomes choisis parmi O, N, P, Si et S;

- R2 et R3, présents sur le même cycle ou chacun sur un cycle différent, représentent, indépendamment l'un de l'autre, un hydrogène, un halogène, ou un groupement de formule -X-G-P (II), sous réserve qu'au moins l'un des radicaux R2 et/ou R3 représente un groupement de formule (II), dans laquelle :

    - X est choisi parmi les groupements -O-, -S-, -SO-, -$SO_2$-, -NH- ou -NR4- avec R4 représentant un radical carboné linéaire, ramifié et/ou cyclique, saturé et/ou insaturé, comprenant 1 à 30 atomes de carbone, éventuellement substitué par un ou plusieurs groupements choisis parmi =O, OH, $NH_2$, et les atomes d'halogènes; et/ou éventuellement interrompu par un ou plusieurs hétéroatomes choisis parmi O, N, P, Si et S;

    - G est un radical carboné divalent linéaire, ramifié et/ou cyclique, saturé et/ou insaturé, comprenant 1 à 32 atomes de carbone, éventuellement substitué par un ou plusieurs groupements choisis parmi =O, OH, $NH_2$, et les atomes d'halogènes; et/ou éventuellement interrompu par un ou plusieurs hétéroatomes choisis parmi O, N, P, Si et S;

- P est un groupement polymérisable choisi parmi l'une des formules suivantes :

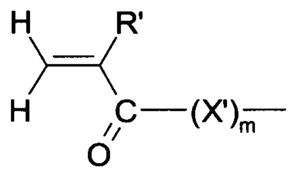

(IIIa)

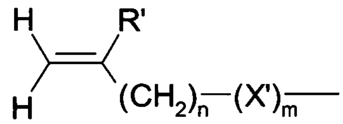

(IIIb)

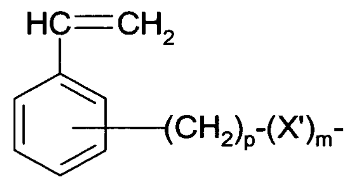

(IIIc)

dans lesquelles :

- R' représente H ou un radical hydrocarboné, linéaire ou ramifié, saturé en C1-6,
- X' représente O, NH ou NR" avec R" représentant un radical choisi parmi les radicaux alkyles en C1-6, aryles en C6-10, aryl(C6-10)alkyles(C1-6) ou alkyle(C1-6)aryles(C6-10), les groupements alkyles et/ou aryles pouvant en outre être substitués par un ou plusieurs groupements choisis parmi OH, halogène, alcoxy en C1-6 et aryloxy en C6-10.
- m est égal à 0 ou 1; n est égal à 0 ou 1 et p est égal à 0, 1 ou 2.

**2.** Polymère selon la revendication 1, dans lequel R1 est un radical hydrocarboné cyclique, linéaire et/ou ramifié, saturé ou insaturé, comprenant éventuellement un cycle hydrocarboné lui-même saturé ou insaturé, comprenant 3 à 18, notamment 4 à 14, atomes de carbone, et peut comprendre au moins un hétéroatome, notamment un ou deux atomes d'azote, d'oxygène ou de silicium.

**3.** Polymère selon l'une des revendications précédentes, dans lequel R1 est un radical hydrocarboné linéaire, ramifié et/ou cyclique, saturé ou insaturé, comprenant 6 à 13 atomes de carbone.

**4.** Polymère selon l'une des revendications précédentes, dans lequel R1 est choisi parmi un radical n-propyle, isopropyle, n-butyle, isobutyle, tert-butyle, n-pentyle, cyclopentyle, n-hexyle, cyclohexyle, n-heptyle, n-octyle, cyclooctyle, décyle, cyclodécyle, dodécyle, cyclododécyle, p-tertbutylcyclohexyle, benzyle, phényle, ou un radical choisi parmi l'éthyl-2-N-pyrrolidine, la (2-méthyl)-1-éthylpyrrolidine, le 3-propyltriéthoxysilane, les dialkylpyrimidines et notamment la 2-(4,6-diméthyl)-pyrimidine, le radical benzothiazyle, le radical fluorényle.

**5.** Polymère selon l'une des revendications précédentes, dans lequel le radical R2 est un atome d'hydrogène et R3 un groupement de formule (II).

**6.** Polymère selon l'une des revendications précédentes, dans lequel dans le groupement de formule (II), X est choisi parmi -O-, -S-, -NH- ou -NR4-.

**7.** Polymère selon l'une des revendications précédentes, dans lequel le radical R4 représente un radical hydrocarboné linéaire, ramifié et/ou cyclique, saturé ou insaturé, comprenant éventuellement un cycle hydrocarboné lui-même saturé ou insaturé, comprenant 2 à 18, notamment 3 à 12 atomes de carbone, éventuellement substitué par un ou plusieurs groupements choisis parmi =O, OH, $NH_2$, et les atomes d'halogènes; et/ou éventuellement interrompu par un ou plusieurs hétéroatomes choisis parmi O, N, P, Si et S; notamment un radical éthyle, n-propyle, isopropyle, n-butyle, isobutyle, tert-butyle, pentyle, hexyle, cyclohexyle, octyle, décyle, dodécyle, phényle ou benzyle.

**8.** Polymère selon l'une des revendications précédentes, dans lequel le radical divalent G est un radical hydrocarboné divalent linéaire, ramifié et/ou cyclique, saturé ou insaturé, comprenant éventuellement un cycle hydrocarboné lui-même saturé ou insaturé, comprenant au total 2 à 18, notamment 3 à 10 atomes de carbone, éventuellement substitué par un ou plusieurs groupements choisis parmi =O, OH, $NH_2$, et les atomes d'halogènes; et/ou éventuellement interrompu par un ou plusieurs hétéroatomes choisis parmi O, N, P, Si.

**9.** Polymère selon l'une des revendications précédentes, dans lequel G est choisi parmi les radicaux hydrocarbonés divalents linéaires ou ramifiés, saturés comprenant éventuellement un cycle hydrocarboné saturé, comprenant au total 2 à 18, notamment 3 à 10 atomes de carbone.

**10.** Polymère selon l'une des revendications précédentes, dans lequel G est choisi parmi les radicaux éthylène, n-propylène, isopropylène (ou méthyl-1 éthylène et méthyl-2 éthylène), n-butylène, isobutylène, pentylène notamment n-pentylène, hexylène notamment n-hexylène, heptylène, cyclohexylène, octylène, décylène, cyclohexyldiméthylène notamment de formule $-CH_2-C_6H_{10}-CH_2-$, dodécylène.

**11.** Polymère selon l'une des revendications précédentes, dans lequel le groupement polymérisable P est choisi parmi l'une des formules suivantes :

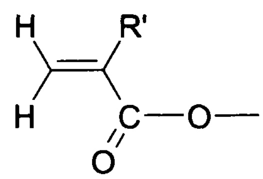

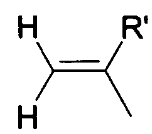

dans lesquelles R' représente H ou méthyle.

**12.** Polymère selon l'une des revendications précédentes, dans lequel le monomère de formule (I) répond à l'une des formules suivantes :

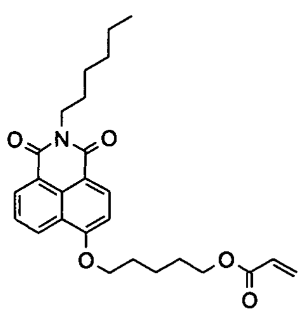

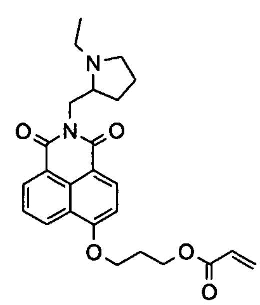

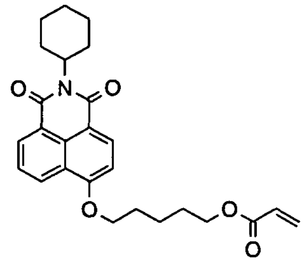

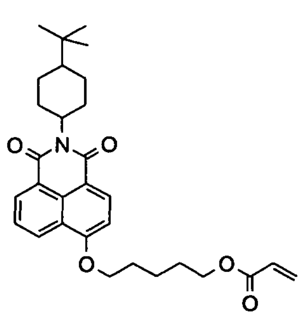

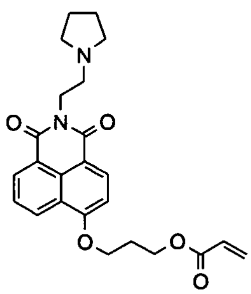

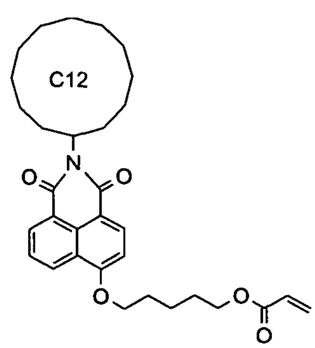

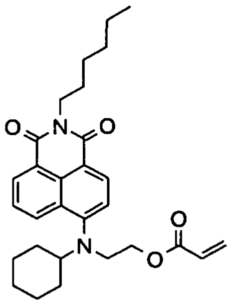
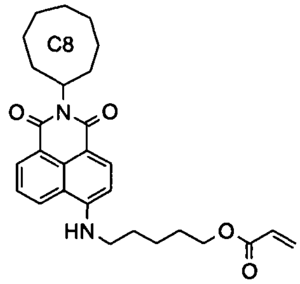
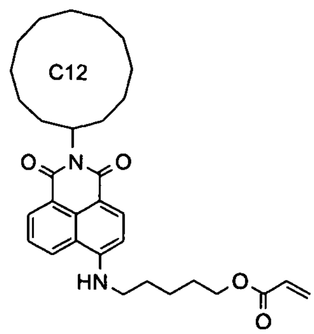

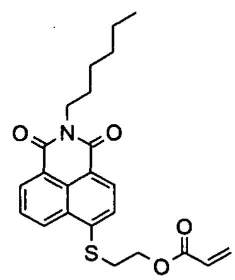
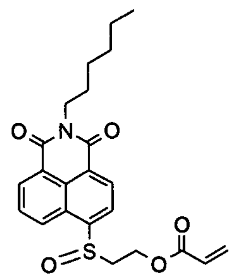

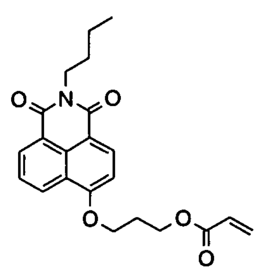
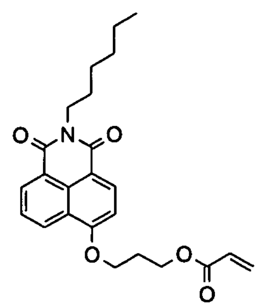
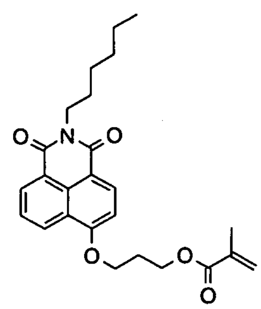

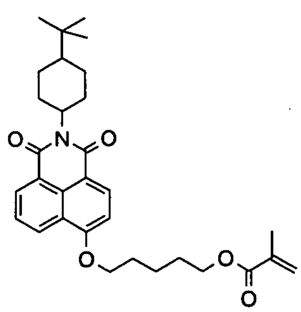
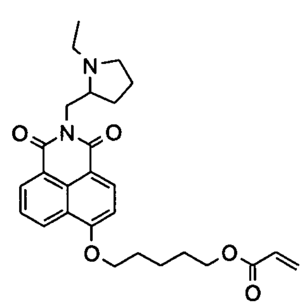
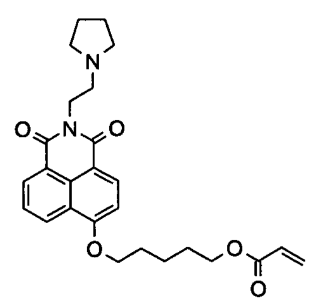

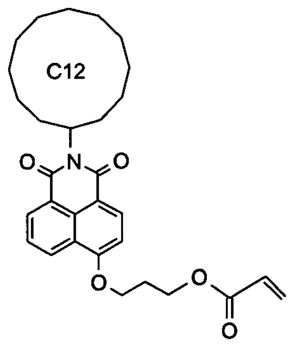

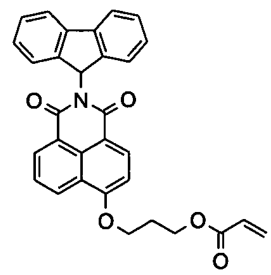

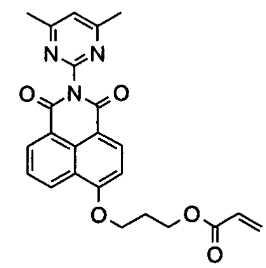

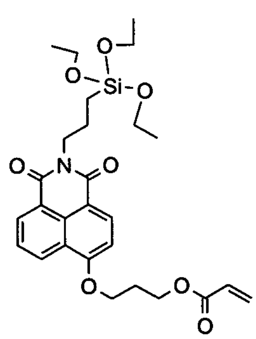

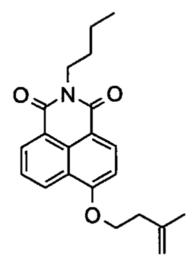

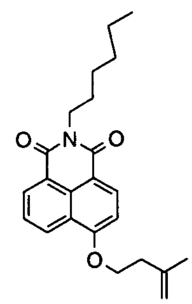

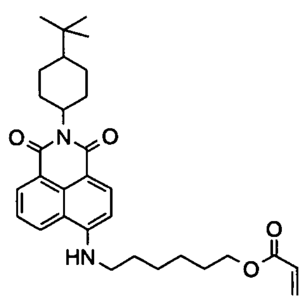

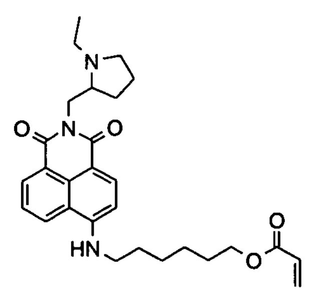

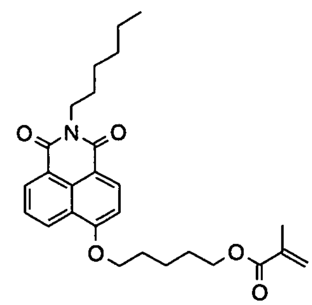

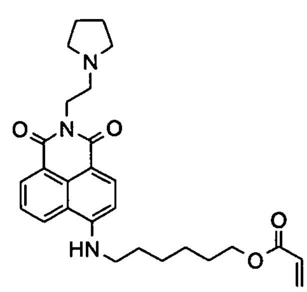

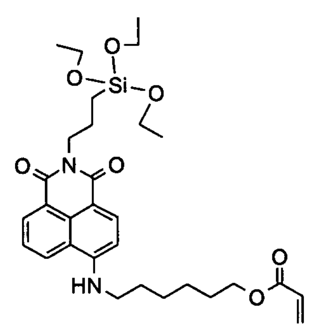

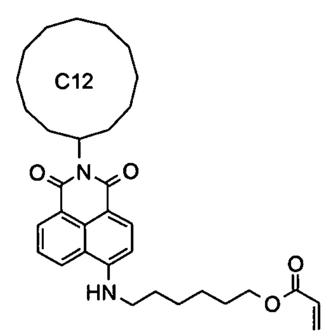

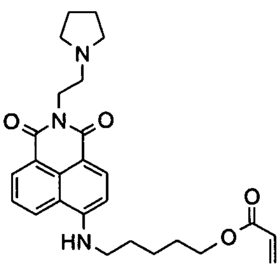

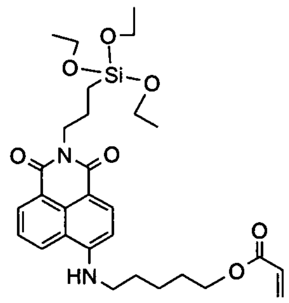

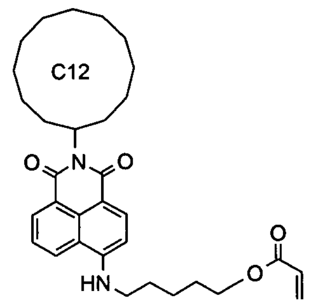

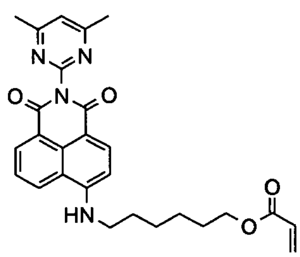

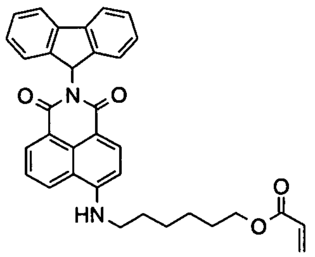

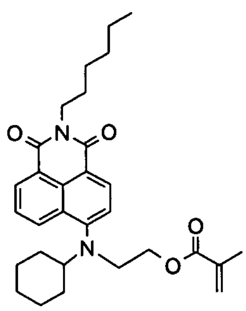

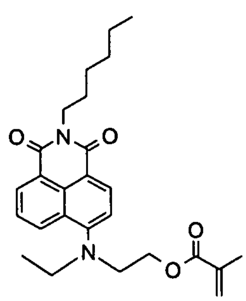

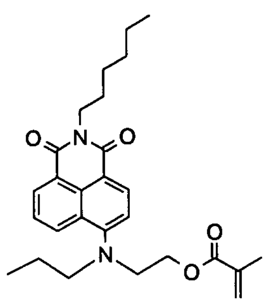

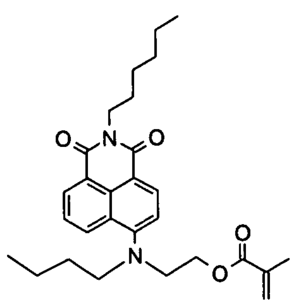

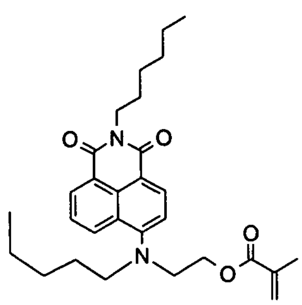

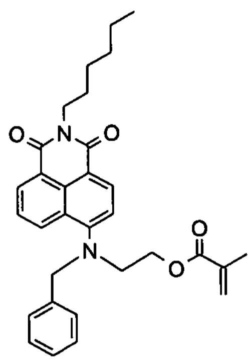
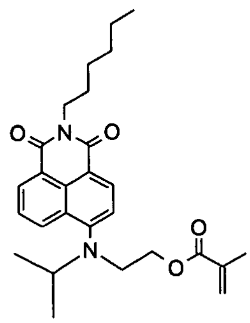
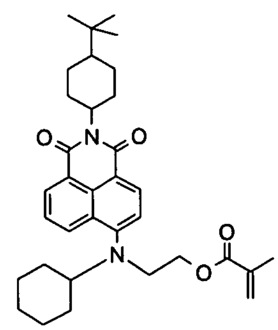

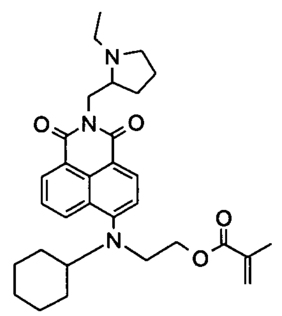
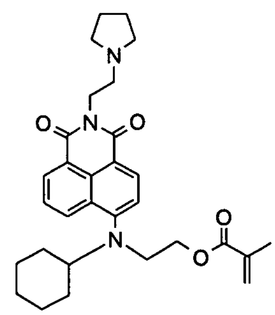
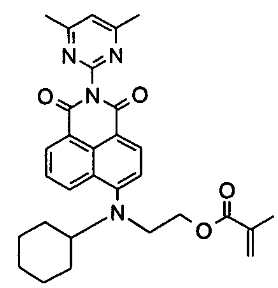

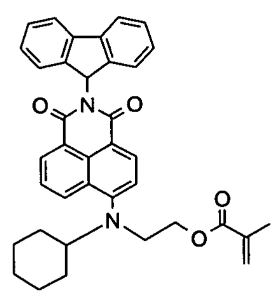
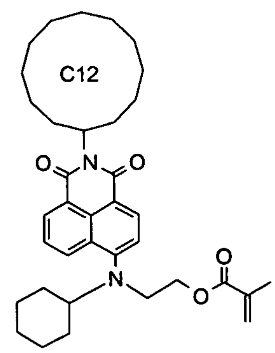

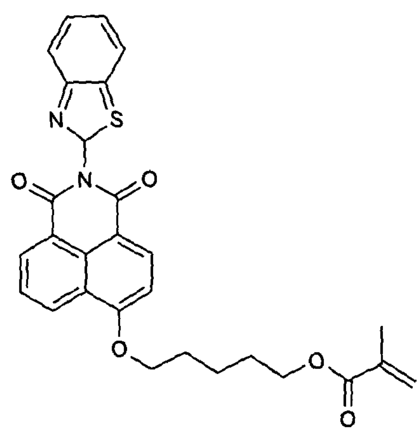
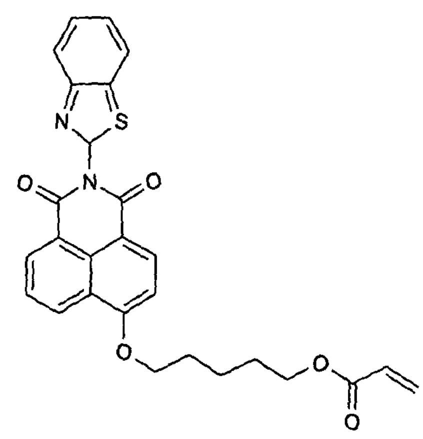

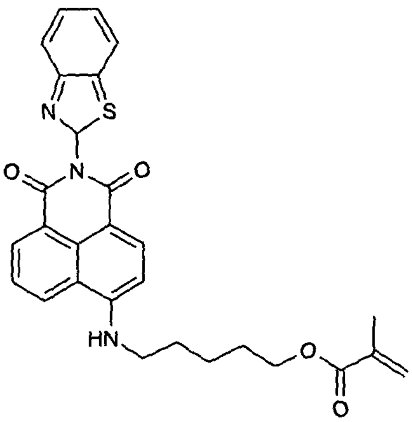

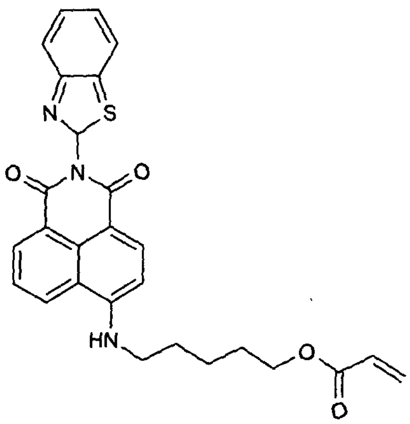

**13.** Polymère selon l'une des revendications précédentes, comprenant par ailleurs au moins un comonomère additionnel choisi parmi, seul ou en mélange, les monomères suivants :

- (i) les hydrocarbures éthyléniques ayant 2 à 10 carbones, tels que l'éthylène, l'isoprène, ou le butadiène ;

- (ii) les (méth)acrylates de formule :

$$CH_2 = CHCOOR'_3$$

ou

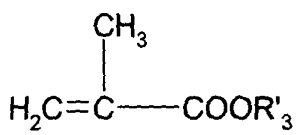

dans lesquelles $R'_3$ représente :

- un groupe alkyle linéaire ou ramifié, de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F), et les groupes Si ($R_4R_5$), où $R_4$ et $R_5$ identiques ou différents représentent un groupe alkyle en $C_1$ à $C_6$ ou un groupe phényle ;
  notamment R'3 peut être un groupe méthyle, éthyle, propyle, n-butyle, isobutyle, tertiobutyle, hexyle, éthylhexyle, octyle, lauryle, isooctyle, isodécyle, dodécyle, cyclohexyle, t-butylcyclohexyle ou stéaryle; éthyl-2-perfluorohexyle; ou un groupe hydroxyalkyle en $C_{1-4}$ tel que 2-hydroxyéthyle, 2-hydroxybutyle et 2-hydroxypropyle; ou un groupe alcoxy ($C_{1-4}$) alkyle ($C_{1-4}$) tel que méthoxyéthyle, éthoxyéthyle et méthoxypropyle,
- un groupe cycloalkyle en $C_3$ à $C_{12}$, tel que le groupe isobornyle,
- un groupe aryle en $C_3$ à $C_{20}$ tel que le groupe phényle,
- un groupe aralkyle en $C_4$ à $C_{30}$ (groupe alkyle en $C_1$ à $C_8$) tel que 2-phényl-éthyle, t-butylbenzyle ou benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle de 1 à 4 C), tel que furfurylméthyle ou tétrahydrofurfurylméthyle,
  lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-4, linéaires ou ramifiés dans lesquels se trouve(nt) éventuellement intercalé(s) un ou plusieurs hétéroatomes choisis parmi O, N, S et P, lesdits groupes alkyle pouvant, en

outre, être éventuellement substitués par un ou plusieurs substituants choisis parmi les groupes hydroxyle, les atomes d'halogène (Cl, Br, I et F), et les groupes Si ($R_4R_5$), où $R_4$ et $R_5$ identiques ou différents représentent un groupe alkyle en $C_1$ à $C_6$, ou un groupe phényle,

- R'$_3$ peut également être un groupe -($C_2H_4O$)$_m$-R", avec m = 5 à 150 et R" = H ou alkyle de $C_1$ à $C_{30}$, par exemple -POE-méthyle ou -POE-béhényle;

- (iii) les (méth)acrylamides de formule :

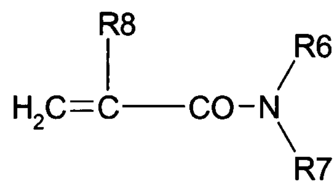

dans laquelle $R_8$ désigne H ou méthyle; et $R_7$ et $R_6$ identiques ou différents représentent :

- un atome d'hydrogène; ou
- un groupe alkyle linéaire ou ramifié, de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F), et les groupes Si ($R_4R_5$), où $R_4$ et $R_5$ identiques ou différents représentent un groupe alkyle en $C_1$ à $C_6$ ou un groupe phényle ; notamment R'3 peut être un groupe méthyle, éthyle, propyle, n-butyle, isobutyle, tertiobutyle, hexyle, éthylhexyle, octyle, lauryle, isooctyle, isodécyle, dodécyle, cyclohexyle, t-butylcyclohexyle ou stéaryle; éthyl-2-perfluorohexyle; ou un groupe hydroxyalkyle en $C_{1-4}$ tel que 2-hydroxyéthyle, 2-hydroxybutyle et 2-hydroxypropyle; ou un groupe alcoxy ($C_{1-4}$) alkyle ($C_{1-4}$) tel que méthoxyéthyle, éthoxyéthyle et méthoxypropyle,
- un groupe cycloalkyle en $C_3$ à $C_{12}$, tel que le groupe isobornyle,
- un groupe aryle en $C_3$ à $C_{20}$ tel que le groupe phényle,
- un groupe aralkyle en $C_4$ à $C_{30}$ (groupe alkyle en $C_1$ à $C_8$) tel que 2-phényl-éthyle, t-butylbenzyle ou benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle de 1 à 4 C), tel que furfurylméthyle ou tétrahydrofurfurylméthyle,

lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle, les atomes d'halogène (Cl, Br, I et F), et les groupes Si ($R_4R_5$), où $R_4$ et $R_5$ identiques ou différents représentent un groupe alkyle en $C_1$ à $C_6$, ou un groupe phényle.

- (iv) les composés vinyliques de formules :

$$CH_2=CH-R_9,\ CH_2=CH-CH_2-R_9$$

ou

$$CH_2=C(CH_3)-CH_2-R_9$$

dans lesquelles $R_9$ est un groupe hydroxyle, halogène (Cl ou F), $NH_2$, $OR_{10}$ où $R_{10}$ représente un groupe phényle ou un groupe alkyle en $C_1$ à $C_{12}$ (le monomère est un éther de vinyle ou d'allyle); acétamide ($NHCOCH_3$); un groupe $OCOR_{11}$ où $R_{11}$ représente un groupe alkyle de 2 à 12 carbones, linéaire ou ramifié (le monomère est un ester de vinyle ou d'allyle) ; ou un groupe choisi parmi :

- un groupe alkyle linéaire ou ramifié, de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F), et les groupes Si ($R_4R_5$), où $R_4$ et $R_5$ identiques ou différents représentent un groupe alkyle en $C_1$ à $C_6$ ou un groupe phényle ;
- un groupe cycloalkyle en $C_3$ à $C_{12}$ tel que isobornyle, cyclohexane,
- un groupe aryle en $C_3$ à $C_{20}$ tel que phényle,
- un groupe aralkyle en $C_4$ à $C_{30}$ (groupe alkyle en $C_1$ à $C_8$) tel que 2-phényléthyle ; benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle de 1 à 4 C), tel que furfurylméthyle ou tétrahydrofurfurylméthyle,

lesdits groupes cycloalkyle, aryle, aralkyle, hétérocyclique ou hétérocycloalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle les atomes d'halogène (Cl, Br, I et F) et les groupes Si($R_4R_5$) où $R_4$ et $R_5$ identiques ou différents représentent un groupe alkyle en $C_1$ à $C_6$, ou un groupe phényle.

- (v) les monomères (méth)acryliques, (méth)acrylamides ou vinyliques à groupe fluoré ou perfluoré, tels que le (méth)acrylate d'éthyl-perfluorooctyle ou d'éthyl-2-perfluorohexyle;

- (vi) les monomères (méth)acryliques, (méth)acrylamides ou vinyliques siliconés, tels que le méthacryloxypropyltris(triméthylsiloxy)silane ou l'acryloxypropylpoly-diméthylsiloxane.

- (vii) les monomères à insaturation(s) éthylénique(s) comprenant au moins une fonction acide carboxylique, phosphorique ou sulfonique, ou anhydride, comme par exemple l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,

- (viii) les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci.

**14.** Polymère selon l'une des revendications précédentes, comprenant par ailleurs au moins un comonomère additionnel choisi parmi, seul ou en mélange, les monomères à effet optique de formule (A), (B) et/ou, (C) :

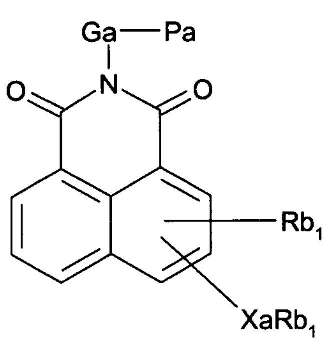

(A)

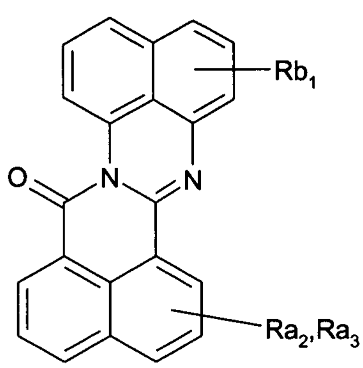

(B)

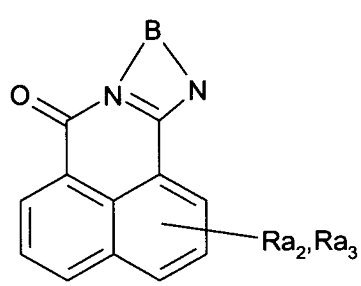

(C)

dans lesquelles :

- Rb1 est choisi parmi (i) un atome d'hydrogène, (ii) un halogène, (iii) un radical carboné linéaire, ramifié et/ou

cyclique, saturé et/ou insaturé, comprenant 1 à 12 atomes de carbone, éventuellement substitué par un ou plusieurs groupements choisis parmi =O, OH, $NH_2$ et/ou éventuellement interrompu par un ou plusieurs hétéroatomes choisis parmi O, N, P, Si et S; (iv) un groupement NRR' avec R et R' étant, indépendamment 'un de l'autre, un atome d'hydrogène ou un radical hydrocarboné, linéaire, cyclique ou ramifié, saturé en C1-6, notamment méthyle, éthyle, propyle, isopropyle, n-butyle, iso-butyle, tert-butyle, pentyle ou hexyle;

- Ra2 et Ra3, présents sur le même cycle ou chacun sur un cycle différent, représentent, indépendamment l'un de l'autre, un hydrogène, un halogène, ou un groupement de formule -Xa-Ga-Pa (II), sous réserve qu'au moins l'un des radicaux Ra2 et/ou Ra3 représente un groupement de formule (II), dans laquelle :

- Xa est choisi parmi les groupements -O-, -S-, -SO-, $-SO_2-$, -NH- ou -NR4- avec R4 représentant un radical carboné linéaire, ramifié et/ou cyclique, saturé et/ou insaturé, comprenant 1 à 30 atomes de carbone, éventuellement substitué par un ou plusieurs groupements choisis parmi =O, OH, $NH_2$, et les atomes d'halogènes; et/ou éventuellement interrompu par un ou plusieurs hétéroatomes choisis parmi O, N, P, Si et S;

- Ga est un radical carboné divalent linéaire, ramifié et/ou cyclique, saturé et/ou insaturé, comprenant 1 à 32 atomes de carbone, éventuellement substitué par un ou plusieurs groupements choisis parmi =O, OH, $NH_2$, et les atomes d'halogènes; et/ou éventuellement interrompu par un ou plusieurs hétéroatomes choisis parmi O, N, P, Si et S;

- Pa est un groupement polymérisable choisi parmi l'une des formules suivantes:

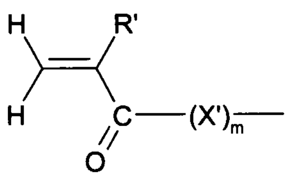
(IIIa)

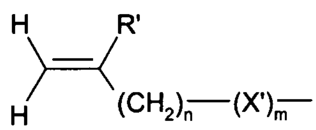
(IIIb)

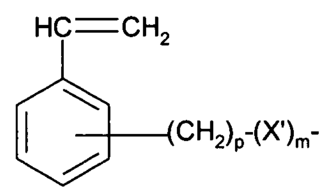
(IIIc)

dans lesquelles :

- R' représente H ou un radical hydrocarboné, linéaire ou ramifié, saturé en C1-6,
- X' représente O, NH ou NR" avec R" représentant un radical choisi parmi les radicaux alkyles en C1-6, aryles en C6-10, aryl(C6-10)alkyles(C1-6) ou alkyle(C1-6)aryles(C6-10), les groupements alkyles et/ou aryles pouvant en outre être substitués par un ou plusieurs groupements choisis parmi OH, halogène, alcoxy en C1-6 et aryloxy en C6-10; et
- m est égal à 0 ou 1;n est égal à 0 ou 1; p est égal à 0, 1 ou 2;

- B représente l'un des groupements aromatiques divalents suivants (IVa) à (IVd) :

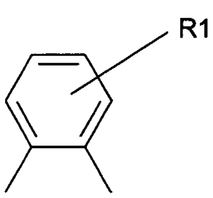
(IVa)

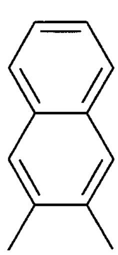
(IVb)

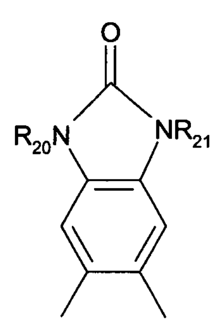
(IVc)

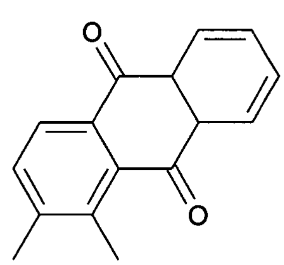
(IVd)

dans lesquels :

- R1 est un radical carboné linéaire, ramifié et/ou cyclique, saturé et/ou insaturé, comprenant 1 à 32 atomes de carbone, éventuellement substitué par un ou plusieurs groupements choisis parmi =O, OH, $NH_2$, et les atomes d'halogènes;
- R20 et R21 sont, indépendamment l'un de l'autre, un atome d'hydrogène, un radical alkyle linéaire ou ramifié en C1-8, un radical cyclopentyle, cyclohexyle, cyclooctyle, cyclodécyle, cyclododécyle, benzyle, naphtyle ou phényle.

**15.** Polymère selon l'une des revendications précédentes, dans lequel le monomère de formule (I), seul ou en mélange, est présent en une quantité de 0,01 à 100% en poids, notamment 0,1 à 99,99% en poids, encore mieux de 0,5 à 70% en poids, voire de 1 à 40% en poids, par exemple de 1,5 à 30% en poids, par rapport au poids de la séquence le/les comprenant.

**16.** Polymère selon l'une des revendications précédentes, dans lequel le monomère de formule (I), seul ou en mélange, est présent en une quantité de 0,01 à 70% en poids, notamment 0,1 à 50% en poids, encore mieux 0,5 à 30% en poids, voire 1 à 20% en poids, par rapport au poids total du polymère.

**17.** Composition notamment cosmétique comprenant, dans un milieu physiologiquement, notamment cosmétiquement, acceptable, au moins un polymère tel que défini à l'une des revendications 1 à 16.

**18.** Composition selon la revendication 17, dans laquelle le polymère est présent, seul ou en mélange, en une quantité de 0,01 à 75% en poids, de préférence 0,1 à 70% en poids, notamment 1 à 65% en poids, voire 3 à 60% en poids, et encore mieux 5 à 50% en poids, préférentiellement 6 à 25% en poids, par rapport au poids total de la composition.

**19.** Composition selon l'une des revendications 17 à 18, dans laquelle le milieu physiologiquement acceptable comprend un milieu hydrophile comprenant de l'eau ou un mélange eau/solvant(s) organique(s) hydrophile(s) et/ou comprend une phase grasse.

**20.** Composition selon l'une des revendications 17 à 19, dans laquelle la phase grasse comprend des cires, corps gras pâteux, gommes, solvants organiques lipophiles, huiles, et/ou de leurs mélanges.

**21.** Composition selon l'une des revendications 17 à 20, comprenant en outre une phase particulaire qui peut comprendre des pigments et/ou des nacres et/ou des charges.

**22.** Composition selon l'une des revendications 17 à 21, comprenant des matières colorantes choisies parmi les colorants hydrosolubles et/ou les colorants liposolubles.

**23.** Composition selon l'une des revendications 17 à 22, comprenant au moins un polymère additionnel tel qu'un polymère filmogène.

**24.** Composition selon l'une des revendications 17 à 23, comprenant au moins un ingrédient choisi parmi les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, ou leurs mélanges.

**25.** Composition selon l'une des revendications 17 à 24, se présentant sous la forme d'une suspension, une dispersion notamment d'huile dans de l'eau grâce à des vésicules; une solution huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; une poudre libre, compacte ou coulée; une pâte anhydre; une lotion, une crème, une pommade, une pâte souple, un onguent, un solide coulé ou moulé et notamment en stick ou en coupelle, ou encore de solide compacté.

**26.** Composition selon l'une des revendications 17 à 25, se présentant sous la forme d'un produit de soin et/ou de maquillage de la peau du corps ou du visage, des lèvres, des ongles, des cils, des sourcils et/ou des cheveux, d'un produit solaire ou autobronzant, d'un produit capillaire pour le soin, le traitement, la mise en forme, le maquillage ou la coloration des cheveux.

**27.** Composition selon l'une des revendications 17 à 26, se présentant sous la forme d'une composition de maquillage, notamment un produit pour le teint tel qu'un fond de teint, un fard à joues ou à paupières; un produit pour les lèvres tel qu'un rouge à lèvres ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux); d'une composition de protection ou de soin de la peau du visage, du cou, des mains ou du corps, notamment une composition anti-rides, une composition hydratante ou traitante; une composition anti-solaire ou de bronzage artificiel; d'un produit capillaire, notamment pour la coloration, le maintien de la coiffure, la mise en forme des cheveux, le soin, le traitement ou le nettoyage des cheveux, telle que des shampooings, des gels, des lotions de mise en plis, des lotions pour le brushing, des compositions de fixation et de coiffage telles que les laques ou spray.

**28.** Procédé de traitement cosmétique, notamment de maquillage ou de soin des matières kératiniques telles que la peau du corps ou du visage, des lèvres, des ongles, des cils, des sourcils et/ou des cheveux, comprenant l'application sur lesdites matières d'une composition cosmétique telle que définie à l'une des revendications 17 à 27.

**29.** Composition de vernis à ongles comprenant au moins un polymère selon l'une des revendications 1 à 16, un ou plusieurs solvants organiques, au moins un polymère filmogène et éventuellement au moins un pigment et/ou au moins un colorant.

**30.** Composition de fond de teint comprenant au moins un polymère selon l'une des revendications 1 à 16, au moins une huile dans une phase grasse, au moins un pigment et éventuellement une phase aqueuse.

**31.** Composition anti-age ou anti-rides comprenant au moins un polymère selon l'une des revendications 1 à 16.

**32.** Utilisation d'au moins un polymère selon l'une des revendications 1 à 16 en tant qu'agent tenseur dans une composition cosmétique, notamment dans une composition antirides.

**33.** Procédé de traitement cosmétique d'une peau ridée, tel que le contour de l'oeil, comprenant une étape consistant à appliquer sur ladite peau une composition cosmétique comprenant, dans un milieu cosmétiquement acceptable, au moins un polymère selon l'une des revendications 1 à 16.

Office européen des brevets

# RAPPORT DE RECHERCHE EUROPEENNE

Numéro de la demande
EP 05 29 0197

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | WO 03/046033 A (CAZENEUVE COLETTE ; LION BERTRAND (FR); OREAL (FR); VICIC MARCO (FR);) 5 juin 2003 (2003-06-05)<br>* le document en entier *<br>----- | 1-33 | C08F293/00<br>C08F220/30<br>C08F220/38 |
| A | DATABASE CHEMABS [Online]<br>CHEMICAL ABSTRACTS SERVICE, COLUMBUS, OHIO, US; 25 juin 2001 (2001-06-25), CHEN, MING-QIANG ET AL: "Syntheses and characterization of co-polymeric fluorescent colors of methyl methacrylate and 1,8-naphthalimides containing polymerizable group"<br>XP002300382<br>extrait de STN<br>Database accession no. 2001:456817<br>* abrégé *<br>& HUADONG LIGONG DAXUE XUEBAO , 27(2), 195-197 CODEN: HLIXEV; ISSN: 1006-3080, 2001,<br>----- | 1 | |
| A | CHENG HU, WEIHONG ZHU, WENQJANG LIN, HE TIAN: "Synthesis and luminescence of novel emitting copolymers"<br>SYNTHETIC METALS,<br>vol. 102, 1999, pages 1129-1130,<br>XP002300381<br>scheme 1<br>----- | 1 | DOMAINES TECHNIQUES RECHERCHES (Int.Cl.7)<br>C08F |
| A | EP 0 728 745 A (BAYER AG)<br>28 août 1996 (1996-08-28)<br>* page 8, ligne 35 - page 11; composé 16 *<br>----- | 1 | |
| A | EP 0 851 017 A (THOMSON CSF)<br>1 juillet 1998 (1998-07-01)<br>* figure 1 *<br>----- | 1 | |

Le présent rapport a été établi pour toutes les revendications

| Lieu de la recherche | Date d'achèvement de la recherche | Examinateur |
|---|---|---|
| Munich | 16 juin 2005 | Wirth, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..................................................................
& : membre de la même famille, document correspondant

1

EPO FORM 1503 03.82 (P04C02)

**ANNEXE AU RAPPORT DE RECHERCHE EUROPEENNE RELATIF A LA DEMANDE DE BREVET EUROPEEN NO.** EP 05 29 0197

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-06-2005

| Document brevet cité au rapport de recherche | Date de publication | Membre(s) de la famille de brevet(s) | Date de publication |
|---|---|---|---|
| WO 03046033 A | 05-06-2003 | FR 2832720 A1 | 30-05-2003 |
| | | AU 2002365297 A1 | 10-06-2003 |
| | | EP 1456262 A1 | 15-09-2004 |
| | | WO 03046033 A1 | 05-06-2003 |
| | | JP 2005510598 T | 21-04-2005 |
| | | US 2005020779 A1 | 27-01-2005 |
| EP 0728745 A | 28-08-1996 | DE 19505941 A1 | 22-08-1996 |
| | | CA 2169713 A1 | 22-08-1996 |
| | | CN 1138036 A | 18-12-1996 |
| | | EP 0728745 A1 | 28-08-1996 |
| | | JP 8259542 A | 08-10-1996 |
| EP 0851017 A | 01-07-1998 | FR 2757531 A1 | 26-06-1998 |
| | | DE 69725832 D1 | 04-12-2003 |
| | | DE 69725832 T2 | 29-07-2004 |
| | | EP 0851017 A1 | 01-07-1998 |
| | | JP 10204427 A | 04-08-1998 |

EPO FORM P0460

Pour tout renseignement concernant cette annexe : voir Journal Officiel de l'Office européen des brevets, No.12/82